# EUROPÄISCHE PATENTSCHRIFT

(11) **EP 2 828 266 B1**
(45) Veröffentlichungstag und Bekanntmachung des Hinweises auf die Patenterteilung: **18.10.2017**
(21) Anmeldenummer: 13707817.6
(22) Anmeldetag: 28.02.2013
(51) Int. Cl.: C07D 493/10

(54) **9,9'-SPIROBIXANTHENDERIVATE FÜR ELEKTROLUMINESZENZVORRICHTUNGEN**
9,9'-SPIRO-BIXANTHENE DERIVATIVES FOR ELECTROLUMINESCENT DEVICES
DÉRIVÉS DE 9,9'-SPIRO-BIXANTHENE POUR DISPOSITIFS ÉLECTROLUMINESCENTS

(30) Priorität: 23.03.2012 EP 12002073
(43) Veröffentlichungstag der Anmeldung: 28.01.2015
(73) Patentinhaber: Merck Patent GmbH, 64293 Darmstadt (DE)
(72) Erfinder: STOESSEL, Philipp, 60487 Frankfurt am Main (DE); MONTENEGRO, Elvira, 69469 Weinheim (DE); BUESING, Arne, 65929 Frankfurt am Main (DE); MUJICA-FERNAUD, Teresa, 64283 Darmstadt (DE); KAISER, Joachim, 64289 Darmstadt (DE)
(86) Internationale Anmeldenummer: PCT/EP2013/000593
(87) Internationale Veröffentlichungsnummer: WO 2013/139431

(56) Entgegenhaltungen:
- EP-A1- 1 341 403
- WO-A1-2010/061315
- WO-A2-2006/028977
- Qi Chen ET AL: "Microporous polymeric microsphere via surfactant-free Suzuki coupling polymerization in a single-phase: Porosity and gas uptake", Polymer, vol. 53, no. 10, 1 April 2012 (2012-04-01) , pages 2032-2037, XP055207580, ISSN: 0032-3861, DOI: 10.1016/j.polymer.2012.03.014

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, jedoch immer noch Verbesserungsbedarf, beispielsweise im Hinblick auf Effizienz, Betriebsspannung und Lebensdauer. Außerdem ist es wünschenswert, dass sich die verwendeten Materialien in hoher Ausbeute und Reinheit synthetisieren lassen.

Die Eigenschaften von phosphoreszierenden OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, Lochtransportmaterialien und Elektronen- bzw. Exzitonenblockiermaterialien von besonderer Bedeutung. Verbesserungen dieser Materialien können somit auch zu deutlichen Verbesserungen der OLED-Eigenschaften führen. Auch für fluoreszierende OLEDs gibt es bei diesen Materialien sowie für Emitter und Matrixmaterialien noch Verbesserungsbedarf.

Gemäß dem Stand der Technik werden unter anderem Carbazolderivate, z. B. gemäß WO 2005/039246, US 2005/0069729, JP 2004/288381, EP 1205527, WO 2008/086851 oder WO 2010/061315, Indolocarbazolderivate, z. B. gemäß WO 2007/063754 oder WO 2008/056746, Indenocarbazolderivate, z. B. gemäß WO 2010/136109, oder Dihydroacridinderivate, z. B. gemäß US 2010/0019658, als Matrixmaterialien für phosphoreszierende Emitter in organischen Elektrolumineszenzvorrichtungen eingesetzt. Hier sind weitere Verbesserungen wünschenswert, ebenso wie in Bezug auf die Effizienz, die Lebensdauer und die thermische Stabilität der Materialien.

Gemäß dem Stand der Technik werden weiterhin Arylaminderivate, insbesondere Triarylaminderivate und Bis(diarylamino)arylderivate als Lochinjektions- und Lochtransportmaterialien eingesetzt, beispielsweise auf Basis von Spirobifluorenen (z. B. gemäß DE 102010045405).

Aufgabe der vorliegenden Erfindung ist die Bereitstellung von Verbindungen, welche sich für den Einsatz in einer fluoreszierenden oder phosphoreszierenden OLED eignen, insbesondere als Matrixmaterial oder als Lochinjektions- oder Lochtransport-/ Elektronenblockiermaterial bzw. Exzitonenblockiermaterial, aber auch als Lochblockiermaterial, Matrix für fluoreszierende Emitter oder als fluoreszierender Emitter. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen. Dabei betreffen die Verbesserungen insbesondere die Lebensdauer, die Effizienz und/oder die Betriebsspannung. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine Verbindung gemäß einer von den Formeln (5a) bis (5u), 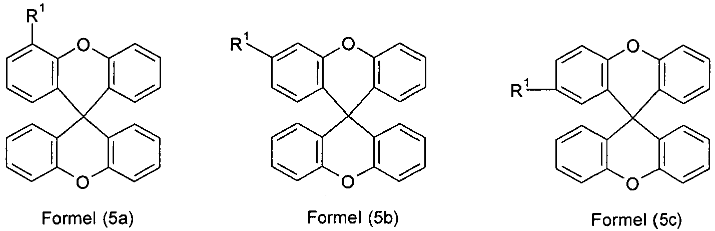 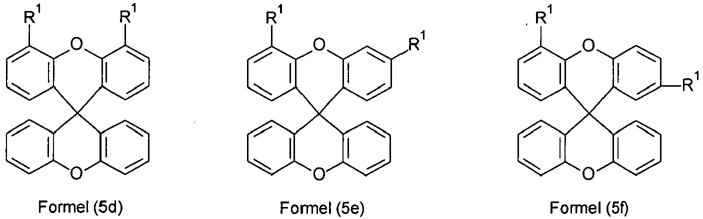 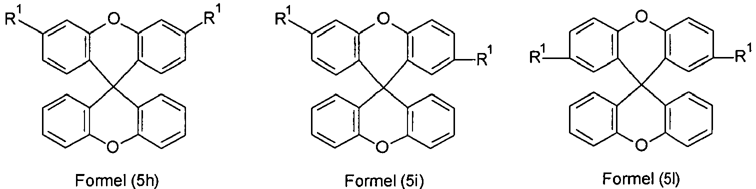 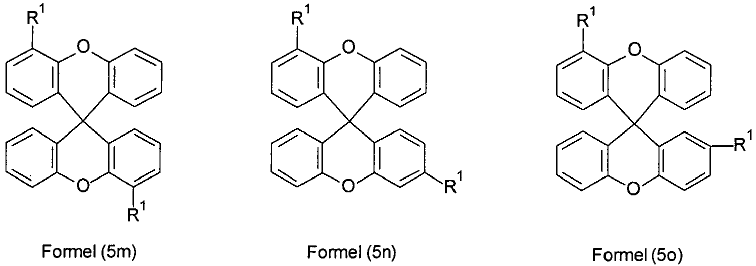 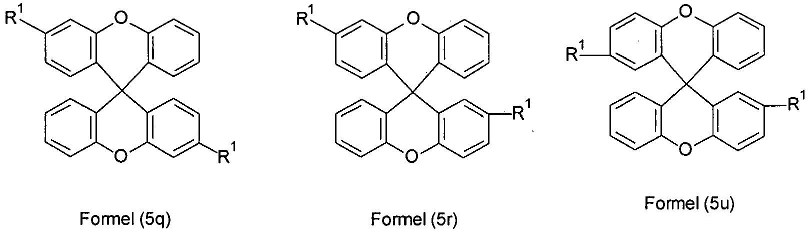 wobei für die verwendeten Symbole und Indizes gilt:
- R¹: ist bei jedem Auftreten gleich oder verschieden ausgewählt aus der Gruppe bestehend aus H, D, F, Cl, Br, I, CN, NO₂, N(Ar¹)₂, N(R³)₂, C(=O)Ar¹, C(=O)R³, P(=O)(Ar¹)₂, B(Ar¹)₂, Si(Ar¹)₃, Si(R³)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C≡C, Si(R³)₂, C=O, C=S, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann;
- Ar¹: ist bei jedem Auftreten gleich oder verschieden ein aromatisches oder heteroaromatisches Ringsystem mit 5-30 aromatischen Ringatomen, das mit einem oder mehreren nicht-aromatischen Resten R³ substituiert sein kann; dabei können zwei Reste Ar¹, welche an dasselbe N-Atom oder P-Atom binden, auch durch eine Einfachbindung oder eine Brücke, ausgewählt aus N(R³), C(R³)₂, O oder S, miteinander verbrückt sein;
- R³: ist bei jedem Auftreten gleich oder verschieden ausgewählt aus der Gruppe bestehend aus H, D, F, Cl, Br, I, CN, NO₂, N(R⁴)₂, C(=O)R⁴, P(=O)(R⁴)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R⁴ substituiert sein kann, wobei eine oder mehrere nicht-benachbarte CH₂-Gruppen durch R⁴C=CR⁴, C≡C, Si(R⁴)₂, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R⁴ substituiert sein kann, einer Aryloxy- oder Heteroaryloxygruppe mit 5 bis 60 aromatischen Ringatomen, die mit einem oder mehreren Resten R⁴ substituiert sein kann, oder einer Kombination dieser Systeme, wobei optional zwei oder mehr benachbarte Substituenten R³ ein monocyclisches oder polycyclisches, aliphatisches Ringsystem bilden können, das mit einem oder mehreren Resten R⁴ substituiert sein kann;
- R⁴: ist bei jedem Auftreten gleich oder verschieden ausgewählt aus der Gruppe bestehend aus H, D, F, CN, einem aliphatischen Kohlenwasserstoffrest mit 1 bis 20 C-Atomen, einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R⁴ miteinander ein mono- oder polycyclisches, aliphatisches Ringsystem bilden können;
mit der Maßgabe, dass in den Formeln (5a) bis (5u) mindestens ein Substituent R¹ vorhanden ist, der ausgewählt ist aus der Gruppe bestehend aus CN, N(Ar¹)₂, C(=O)Ar¹, P(=O)(Ar¹)₂, B(Ar¹)₂, Si(Ar¹)₃, Si(R³)₃ oder einem aromatischen oder heteroaromatischen Ringsystem mit 5 bis 60 aromatischen Ringatomen, das jeweils mit einem oder mehreren Resten R³ substituiert sein kann.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R² oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.

In einer Ausführungsform der Erfindung sind Verbindungen der Formel (1) von der Erfindung ausgenommen, in denen R¹ gleich oder verschieden für Carbazol oder für ein substituiertes Carbazol, das jeweils über das Stickstoffatom an das Grundgerüst gebunden ist, oder für Diphenylamin oder für ein substituiertes Diphenylamin steht.

Besonders bevorzugte Strukturen sind die Strukturen der oben genannten Formeln (5a), (5b), (5c), (5d), (5h), (5l), (5m) und (5u).

Besonders bevorzugt sind auch Strukturen der oben genannten Formeln, die zwei Gruppen R¹ aufweisen, wobei eine Gruppe R¹ eine lochtransportierende Einheit ist und die andere Gruppe R¹ eine elektronentransportierende Einheit. Dabei handelt es sich bei der lochtransportierenden Einheit R¹ insbesondere um eine gegebenenfalls substituierte Carbazolgruppe oder ein Derivat davon oder um eine gegebenenfalls substituierte Diarylaminogruppe oder Triarylaminogruppe. Bei der elektronentransportierenden Einheit R¹ handelt es sich insbesondere um eine gegebenenfalls substituierte elektronenarme Heteroarylgruppe, insbesondere um ein gegebenenfalls substituiertes Triazin oder Pyrimidin, um ein aromatisches Keton -C(=O)Ar¹ oder um ein aromatisches Phosphinoxid -P(=O)(Ar¹)₂.

In einer bevorzugten Ausführungsform der Erfindung ist R¹ in den oben genannten Formeln gleich oder verschieden bei jedem Auftreten ausgewählt aus der Gruppe bestehend aus H, D, F, Cl, Br, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenyl- oder Alkinylgruppe mit 2 bis 10 C-Atomen, die jeweils mit einem oder mehreren Resten R³ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 30 aromatischen Ringatomen, das jeweils mit einem oder mehreren Resten R³ substituiert sein kann.

In einer besonders bevorzugten Ausführungsform der Erfindung ist R¹ in den oben genannten Formeln gleich oder verschieden bei jedem Auftreten ausgewählt aus der Gruppe bestehend aus H, D, F, Cl, Br, CN, einer geradkettigen Alkylgruppe mit 1 bis 5 C-Atomen oder einer verzweigten oder cyclischen Alkylgruppe mit 3 bis 10 C-Atomen, wobei ein oder mehrere H-Atome durch D oder F ersetzt sein können, einem aromatischen oder heteroaromatischen Ringsystem mit 5 bis 18 aromatischen Ringatomen, das jeweils mit einem oder mehreren Resten R³ substituiert sein kann.

Dabei haben für Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn R¹ -für ein aromatisches oder heteroaromatisches Ringsystem steht, ist dieses gleich oder verschieden bei jedem Auftreten bevorzugt aus aromatischen oder heteroaromatischen Ringsvstemen mit 5 bis 24 aromatischen Ringatomen ausgewählt, welche jeweils durch einen oder mehrere Reste R³ substituiert sein können. Besonders bevorzugte Gruppen R¹ sind ausgewählt aus Benzol, ortho-, meta- oder para-Biphenyl, ortho-, meta-, para- oder verzweigtes Terphenyl, ortho-, meta- para- oder verzweigtes Quaterphenyl. 1- oder 2-Naphthyl, Pyrrol, Furan, Thiophen, Indol, Benzofuran, Benzothiophen, Carbazol, Dibenzofuran, Dibenzothiophen, Pyridin, Pyrimidin, Pyrazin, Pyridazin, Triazin, Anthracen, Phenanthren, Pyren, Benzanthracen oder Kombinationen aus zwei oder drei dieser Gruppen, welche jeweils mit einem oder mehreren Resten R³ substituiert sein können.

Wenn die Verbindungen der Formeln (5a) bis (5u) bzw. die bevorzugten Ausführungsformen als Elektronentransportmaterial verwendet werden, ist es bevorzugt, wenn mindestens einer der Reste R¹ für ein elektronenarmes heteroaromatisches Ringsystem oder -C(=O)Ar¹ oder -P(=O)(Ar¹)₂ steht. Elektronenarme Heteroaromaten sind erfindungsgemäß Fünfringheteroaromaten mit mindestens zwei Heteroatomen oder Sechsringheteroaromaten, an die jeweils noch ein oder mehrere aromatische oder heteroaromatische Gruppen ankondensiert sein können, zum Beispiel substituierte oder unsubstituierte Imidazole, Pyrazole, Thiazole, Oxazole, Oxadiazole, Triazole, Pyridine, Pyrazine, Pyrimidine, Pyridazine, Triazine, Benzimidazole, etc., insbesondere solche, wie sie im Folgenden aufgeführt sind.

Wenn die erfindungsgemäße Verbindung als Matrixmaterial für einen phosphoreszierenden Emitter oder als Elektronentransportmaterial eingesetzt wird, ist bevorzugt mindestens ein Substituent R¹ eine elektronenarme Gruppe, insbesondere ausgewählt aus Strukturen gemäß den folgenden Formeln (13) bis (16) 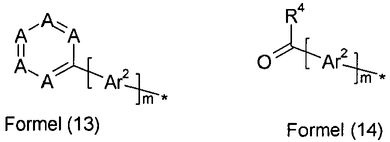 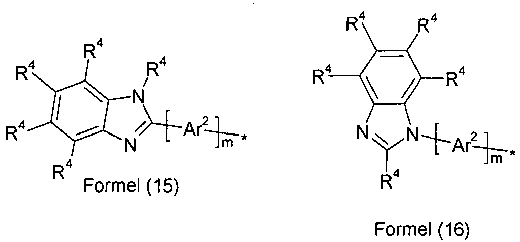 wobei R⁴ die oben genannte Bedeutung haben, * die Position der Bindung der Gruppe gemäß Formel (13) bis (16) andeutet und weiterhin gilt:
- m: ist 0 oder 1;
- A: ist bei jedem Auftreten gleich oder verschieden CR⁴ oder N, mit der Maßgabe, dass eine, zwei oder drei Gruppen A für N stehen;
- Ar²: ist gleich oder verschieden bei jedem Auftreten ein bivalentes aromatisches oder heteroaromatisches Ringsystem mit 5 bis 16 C-Atomen, welches durch einen oder mehrere Reste R⁴ substituiert sein kann.

In einer besonders bevorzugten Ausführungsform der Erfindung steht mindestens ein Substituent R¹ für eine Gruppe der oben genannten Formel (13), wobei jeweils zwei oder drei Symbole A für N stehen und die anderen Symbole A für CR⁴ stehen. Besonders bevorzugte Gruppen R¹ sind daher die Gruppen der folgenden Formeln (20) bis (26), 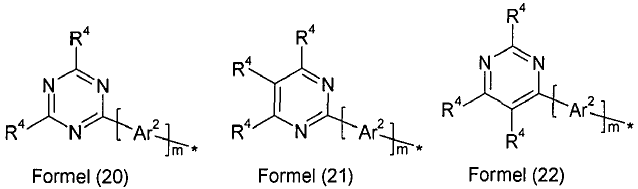 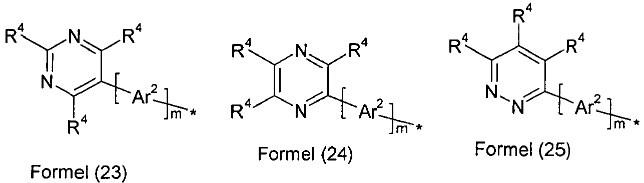 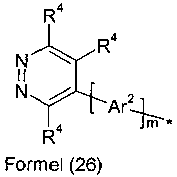 wobei die verwendeten Symbole und Indizes die oben genannten Bedeutungen aufweisen.

Wenn R¹ für eine Gruppe der Formel (20) steht, dann steht R⁴ in dieser Gruppe bevorzugt für ein aromatisches oder heteroaromatisches Ringsystem mit 5 bis 24 aromatischen Ringatomen, welches durch einen oder mehrere Reste R⁵ substituiert sein kann, insbesondere für Phenyl, ortho-, meta- oder para-Biphenyl, ortho-, meta-, para- oder verzweigtes Terphenyl oder ortho-, meta-, para- oder verzweigtes Quaterphenyl.

Wenn R¹ für eine Gruppe der Formel (21) bis (34) steht, dann steht R⁴ in diesen Gruppen bevorzugt gleich oder verschieden bei jedem Auftreten für H, D oder ein aromatisches oder heteroaromatisches Ringsystem mit 5 bis 24 aromatischen Ringatomen, welches durch einen oder mehrere Reste R⁵ substituiert sein kann, insbesondere für H oder Phenyl, ortho-, meta- oder para-Biphenyl, ortho-, meta-, para- oder verzweigtes Terphenyl oder ortho-, meta-, para- oder verzweigtes Quaterphenyl.

Wenn die erfindungsgemäße Verbindung als Matrixmaterial für einen phosphoreszierenden Emitter, als Lochtransportmaterial oder als Elektronen- bzw. Exzitonenblockiermaterial eingesetzt wird, ist mindestens ein Substituent R¹ bevorzugt ausgewählt aus der Gruppe bestehend aus Triarylaminderivaten, Carbazolderivaten, Indenocarbazolderivaten, Indolocarbazolderivaten, Azacarbazolderivaten, Indolderivaten, Furanderivaten, Benzofuranderivaten, Dibenzofuranderivaten, Thiophenderivaten, Benzothiophenderivaten oder Dibenzothiophenderivaten, welche jeweils durch einen oder mehrere Reste R³ substituiert sein können, oder mindestens ein Substituent R¹ -steht für -N(Ar¹)₂. Diese Gruppen sind bevorzugt ausgewählt aus den Gruppen der folgenden Formeln (35) bis (49), 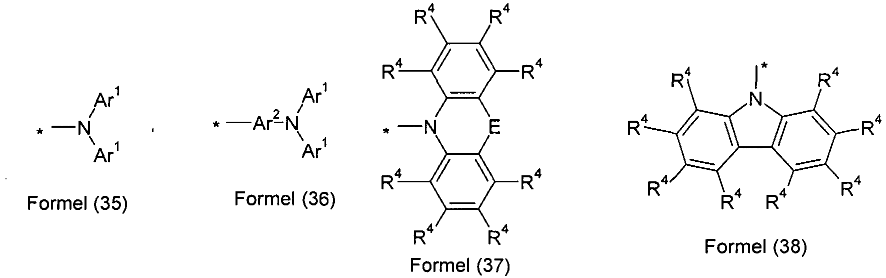 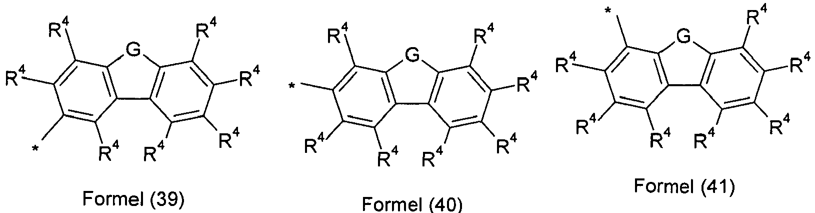 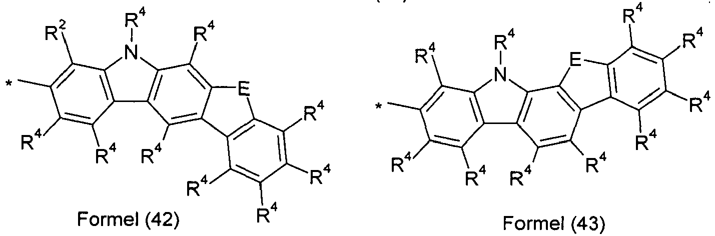 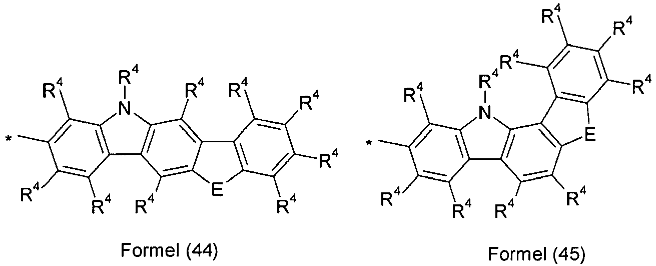 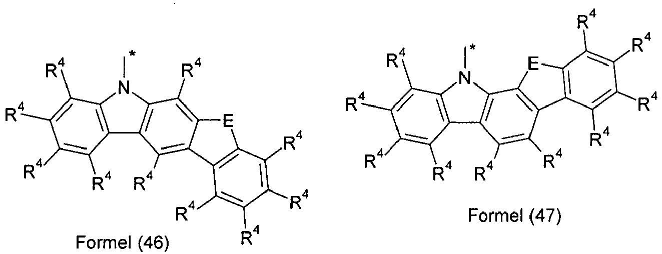 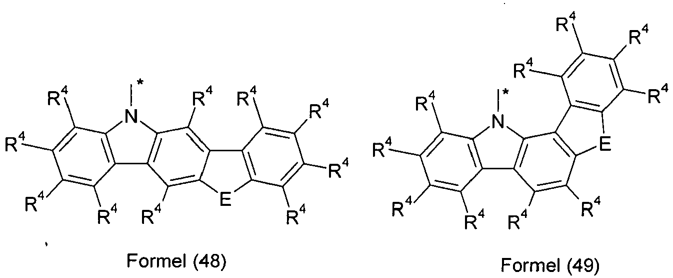 wobei die verwendeten Symbole die oben genannten Bedeutungen aufweisen und weiterhin gilt:
- E: ist ausgewählt aus der Gruppe bestehend aus C(R³)₂, NR³, O oder S;
- G: ist ausgewählt aus der Gruppe bestehend aus NR³, O oder S.

In einer weiteren bevorzugten Ausführungsform der Erfindung stehen in den erfindungsgemäßen Verbindungen die Symbole R¹, die nicht für eine Gruppe der oben aufgeführten Formeln (13) bis (49) stehen, für H oder D.

Die oben genannten bevorzugten Ausführungsformen können beliebig miteinander kombiniert werden. In einer besonders bevorzugten Ausführungsform der Erfindung treten die oben genannten Bevorzugungen gleichzeitig auf.

Wenn die Verbindungen der Formeln (5a) bis (5u) bzw. die bevorzugten Ausführungsformen als Matrixmaterial für einen phosphoreszierenden Emitter verwendet werden, ist es bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, wenn die Reste R¹ keine kondensierte Aryl- bzw. Heteroarylgruppe enthalten, in der zwei oder mehr Sechsringe direkt aneinander ankondensiert sind. Besonders bevorzugt enhält die Verbindung der Formel (1) überhaupt keine kondensierten Aryl- oder Heteroarylgruppen, in denen Sechsringe direkt aneinander ankondensiert sind.

Wenn die Verbindungen der Formeln (5a) bis (5u) bzw. die bevorzugten Ausführungsformen als Matrixmaterial für einen fluoreszierenden Emitter oder als fluoreszierender Emitter verwendet werden, ist es bevorzugt, wenn mindestens einer der Reste R¹ -eine Gruppe enthält, die ausgewählt ist aus Naphthalin, Anthracen, Phenanthren, Pyren und/oder Benzanthracen, welches jeweils noch durch die oben genannten Gruppen substituiert sein kann.

Beispiele für bevorzugte Verbindungen gemäß den oben aufgeführten Ausführungsformen sind die Verbindungen der folgenden Strukturen 1 bis 109.

| | |
|---|---|
| 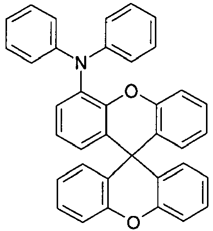 | 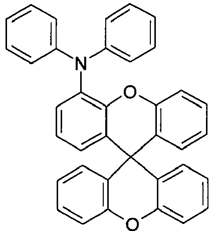 |
| 1* | 2* |
| 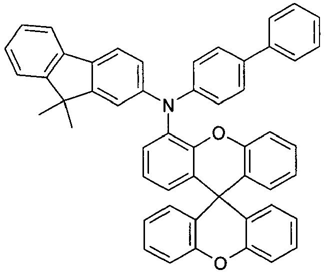 | 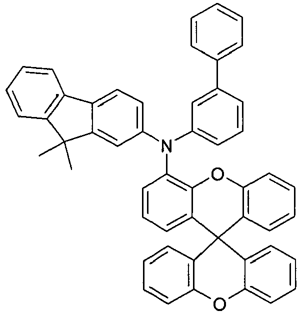 |
| 3* | 4* |
| 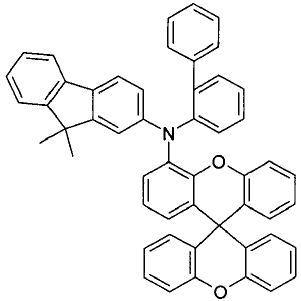 | 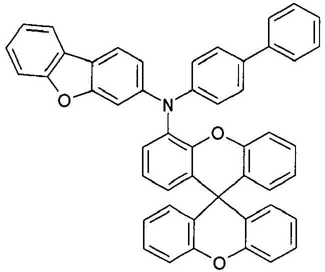 |
| 5* | 6* |
| 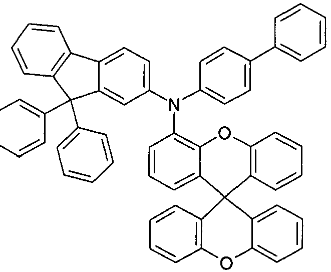 | 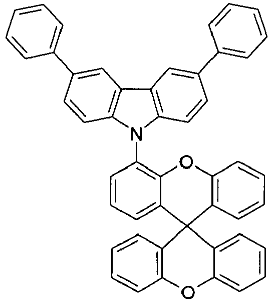 |
| 7* | 8* |
| 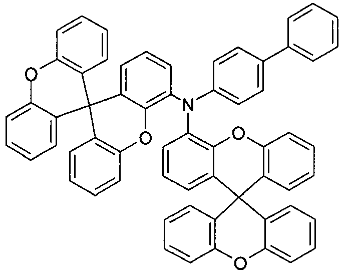 | 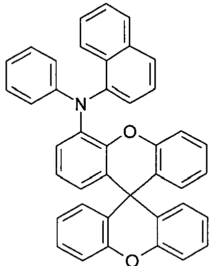 |
| 9* | 10* |
| 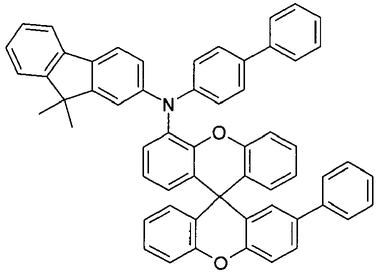 | 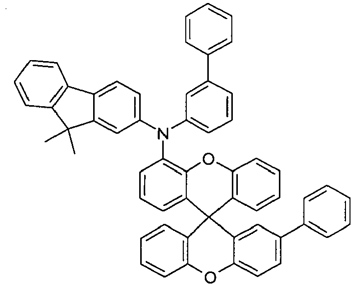 |
| 11* | 12* |
| 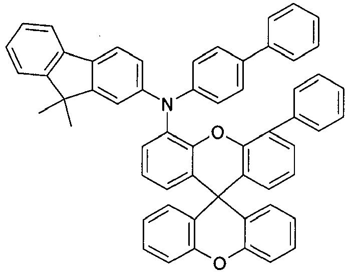 | 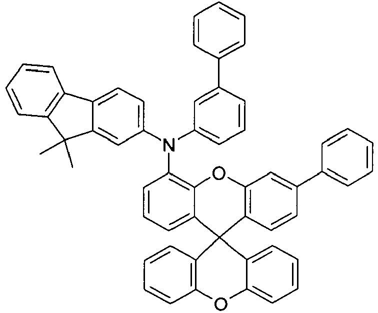 |
| 13* | 14* |
| 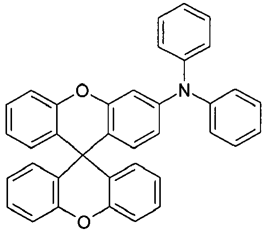 | 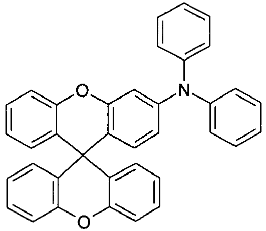 |
| 15* | 16* |
| 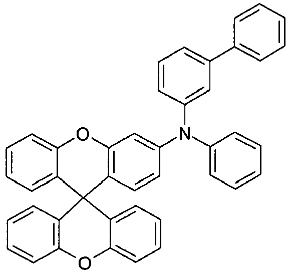 | 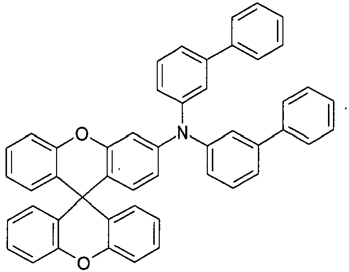 |
| 17* | 18* |
| 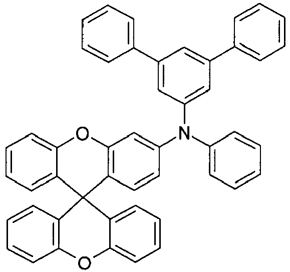 | 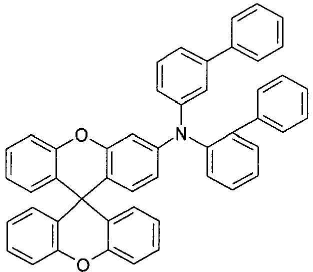 |
| 19* | 20* |
| 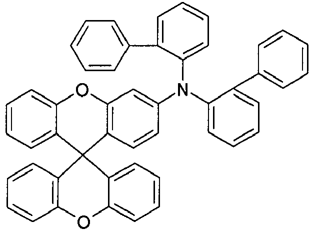 | 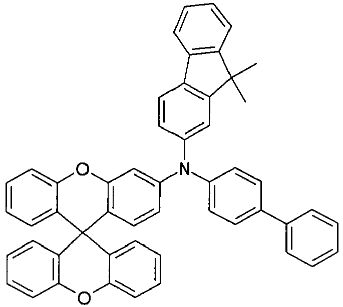 |
| 21* | 22* |
| 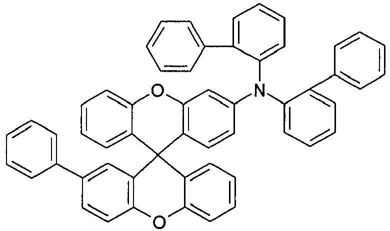 | 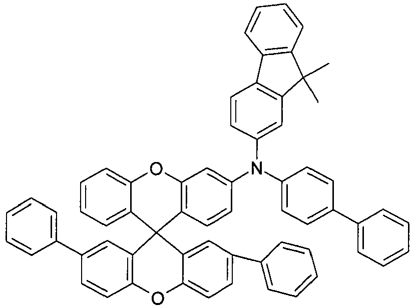 |
| 23* | 24 |
| 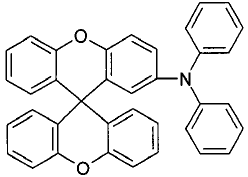 | 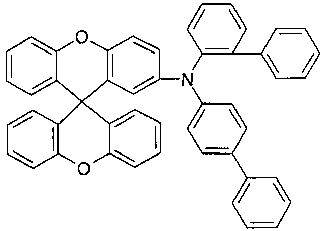 |
| 25* | 26* |
| 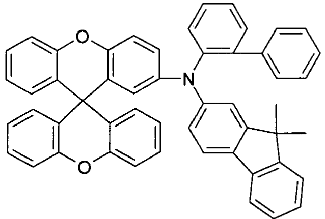 | 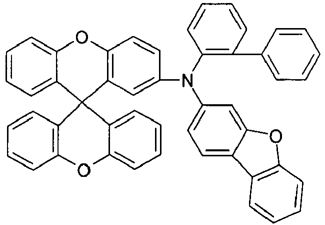 |
| 27* | 28* |
| 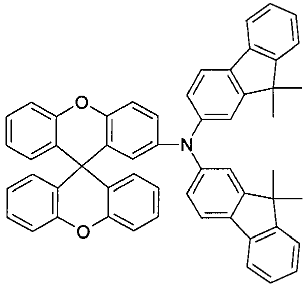 | 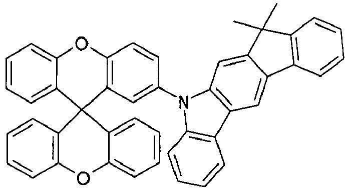 |
| 29* | 30* |
| 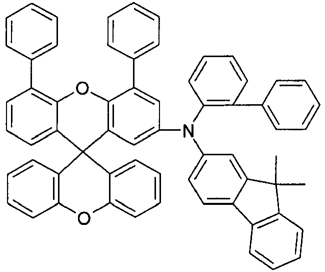 | 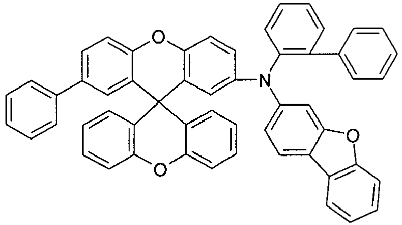 |
| 31 | 32* |
| 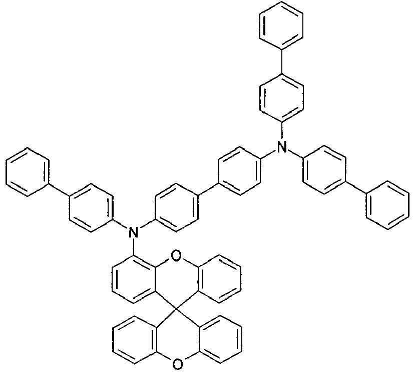 | 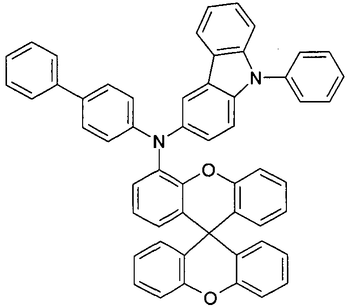 |
| 33* | 34* |
| 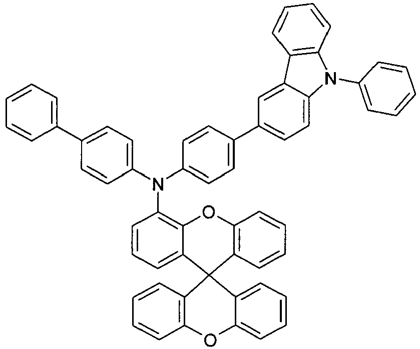 | 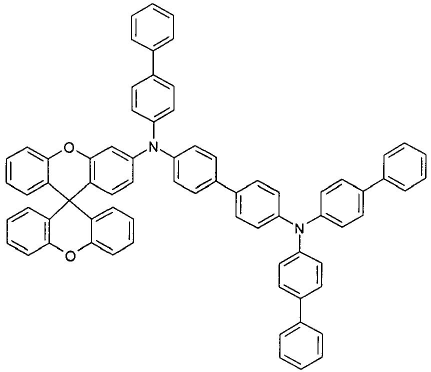 |
| 35* | 36* |
| 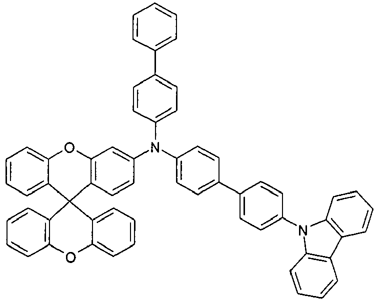 | 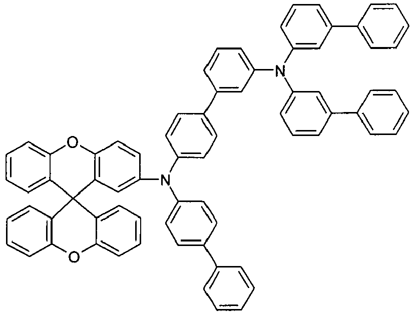 |
| 37* | 38* |
| 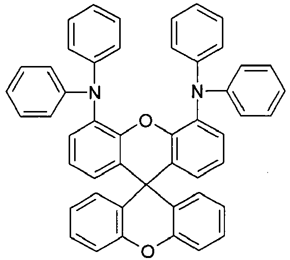 | 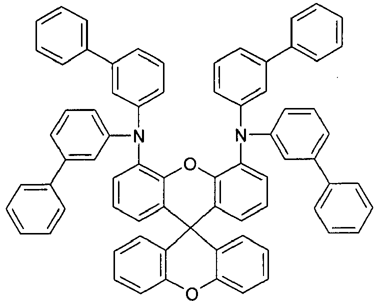 |
| 39* | 40* |
| 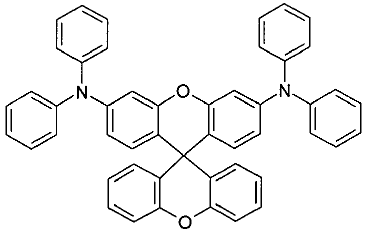 | 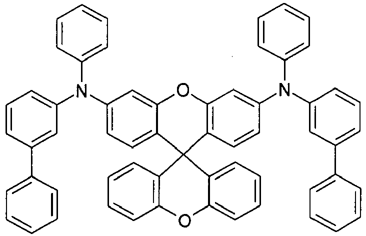 |
| 41* | 42* |
| 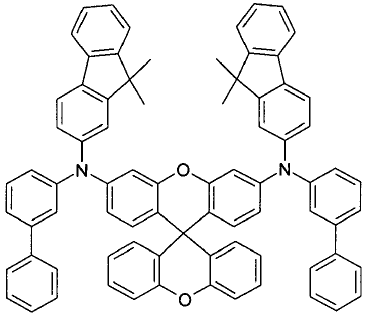 | 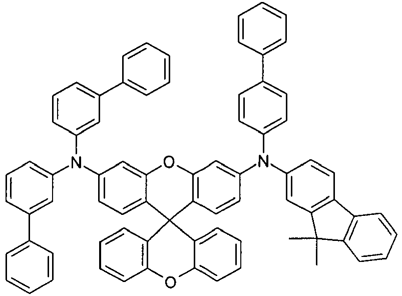 |
| 43* | 44* |
| 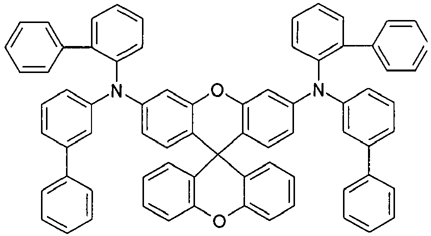 | 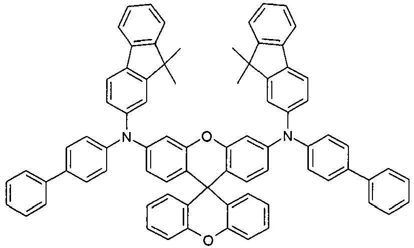 |
| 45* | 46* |
| 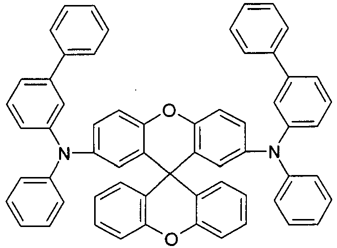 | 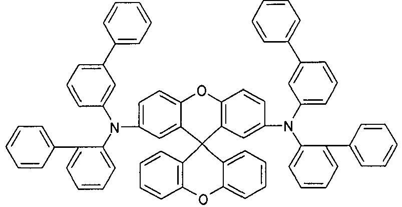 |
| 47* | 48* |
| 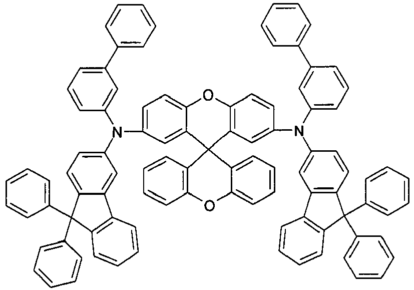 | 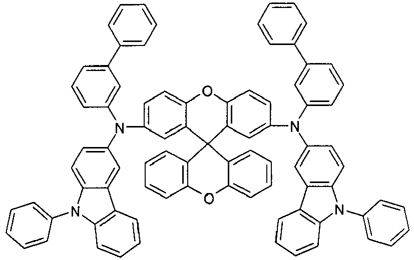 |
| 49* | 50* |
| 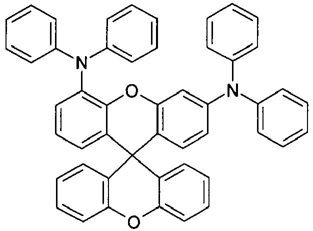 | 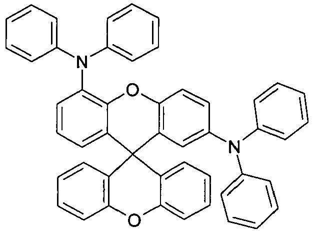 |
| 51* | 52* |
| 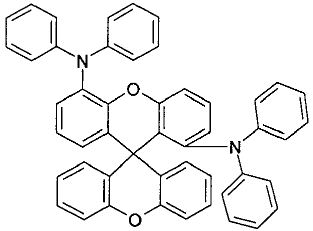 | 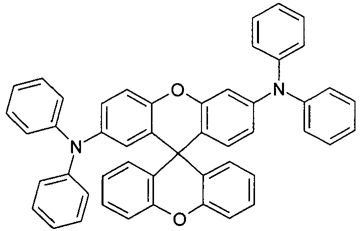 |
| 53* | 54* |
| 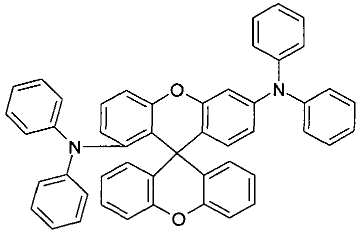 | 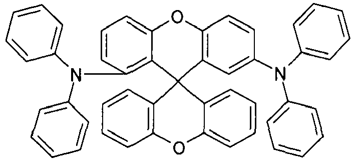 |
| 55* | 56* |
| 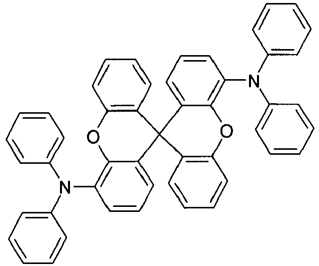 | 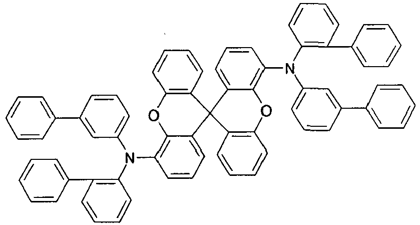 |
| 57* | 58* |
| 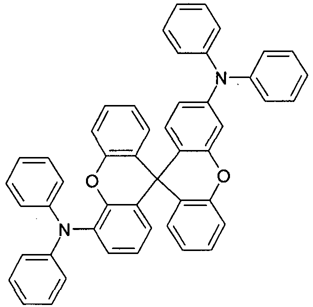 | 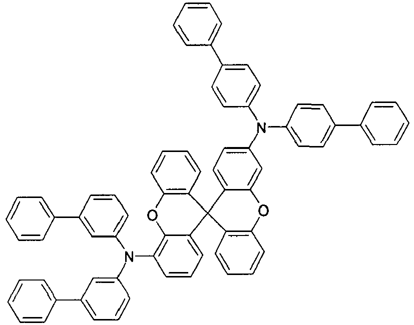 |
| 59* | 60* |
| 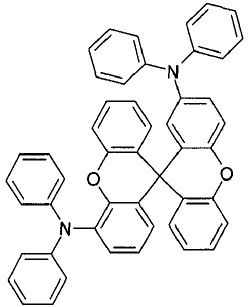 | 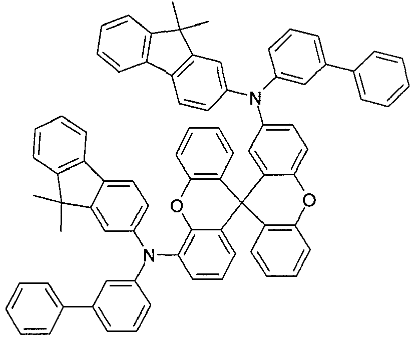 |
| 61* | 62* |
| 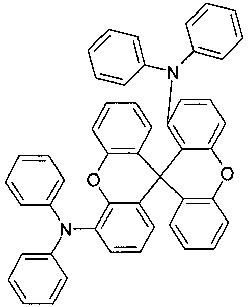 | 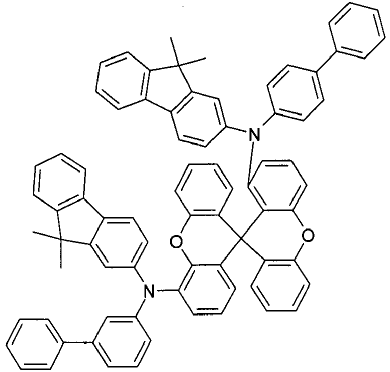 |
| 63* | 64* |
| 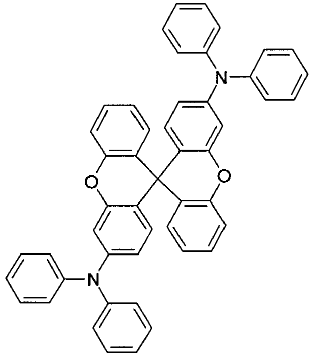 | 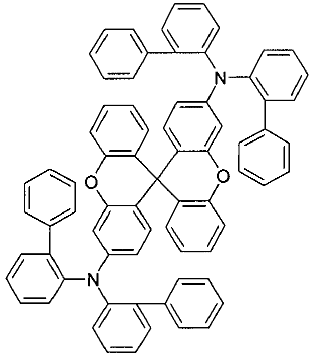 |
| 65* | 66* |
| 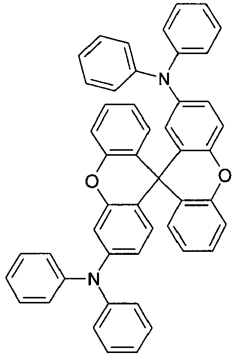 | 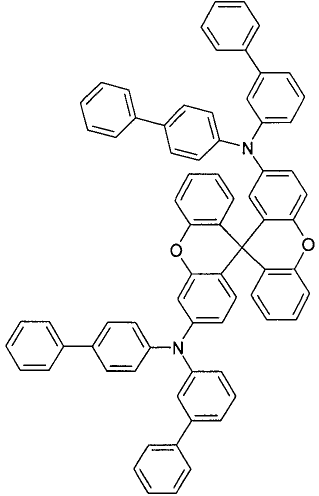 |
| 67* | 68* |
| 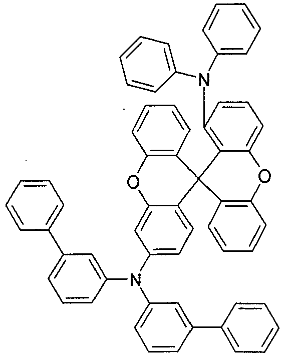 | 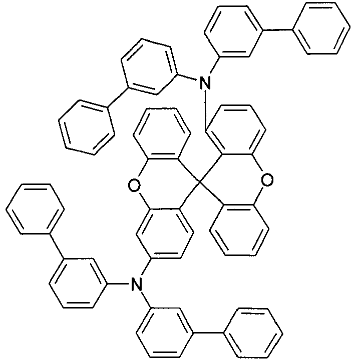 |
| 69* | 70* |
| 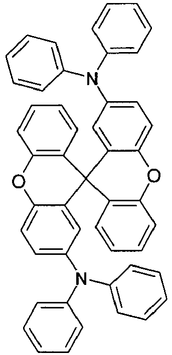 | 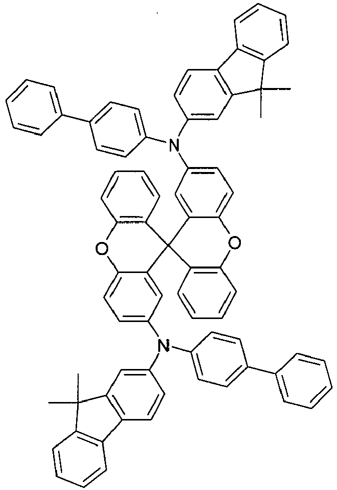 |
| 71* | 72* |
| 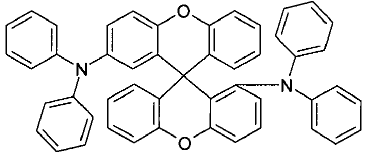 | 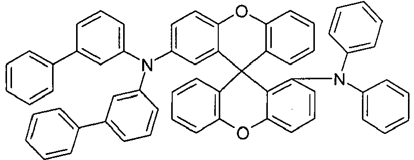 |
| 73* | 74* |
| 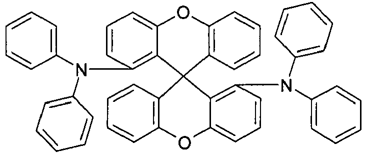 | 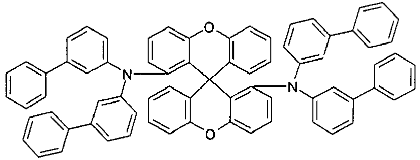 |
| 75* | 76* |
| 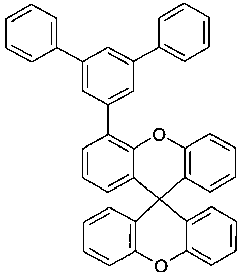 | 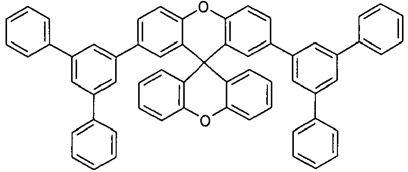 |
| 77* | 78* |
| 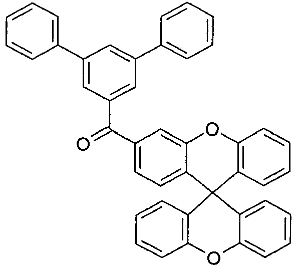 | 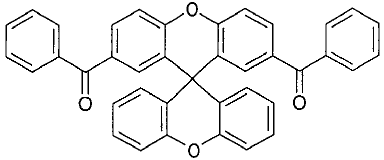 |
| 79* | 80* |
| 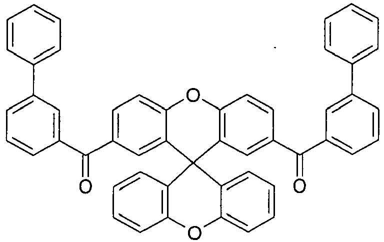 | 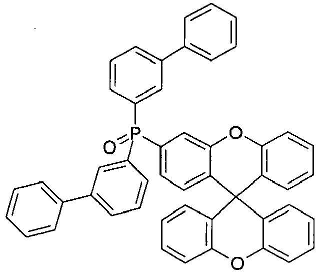 |
| 81* | 82* |
| 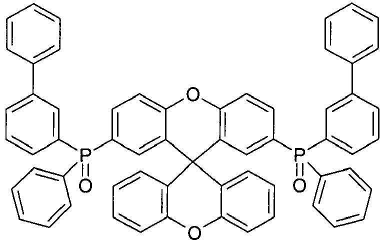 | 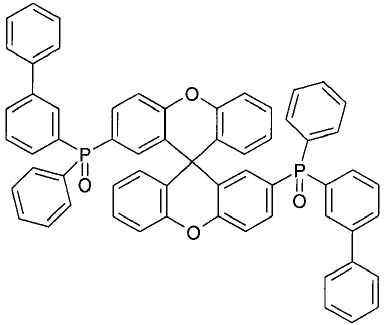 |
| 83* | 84* |
| 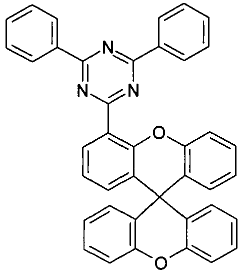 | 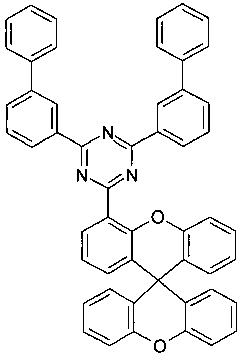 |
| 86* | 86* |
| 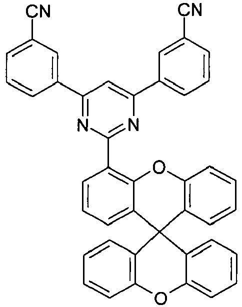 | 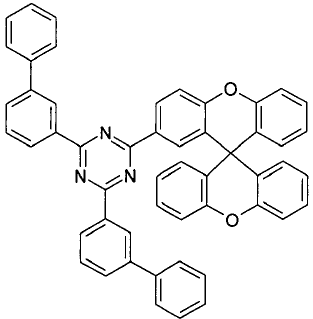 |
| 87* | 88* |
| 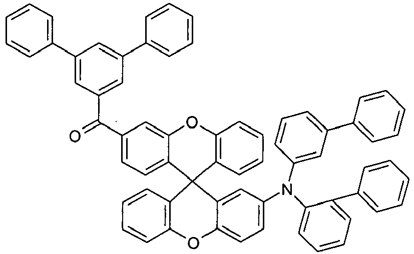 | 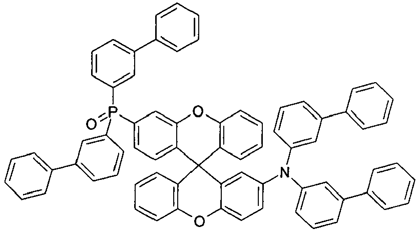 |
| 89* | 90* |
| 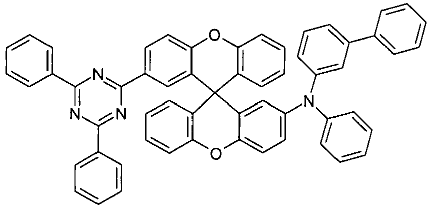 | 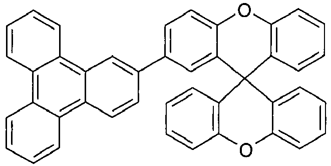 |
| 91* | 92* |
| 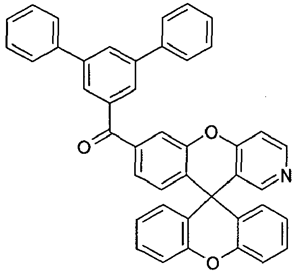 | 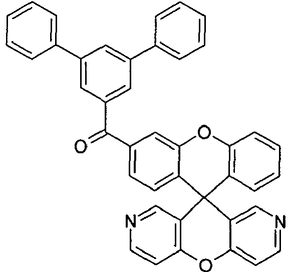 |
| 93 | 94 |
| 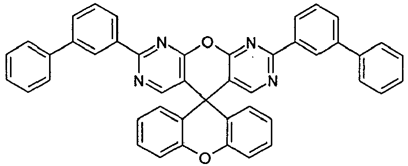 | 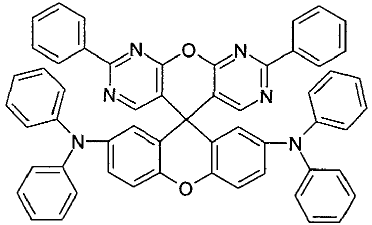 |
| 95 | 96 |
| 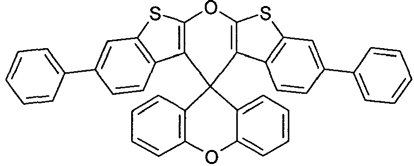 | 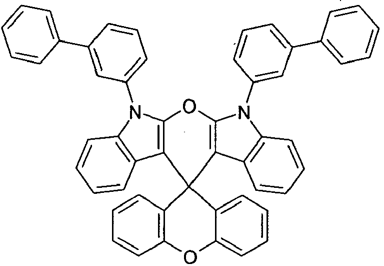 |
| 97 | 98 |
| 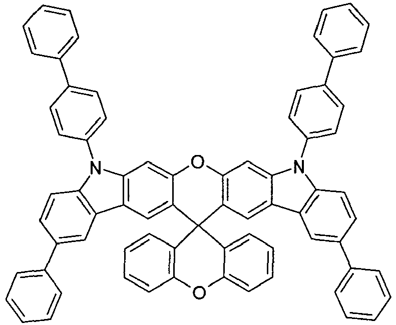 | 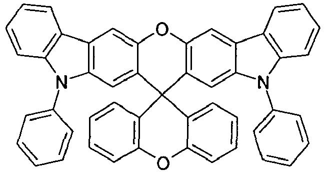 |
| 99 | 100 |
| 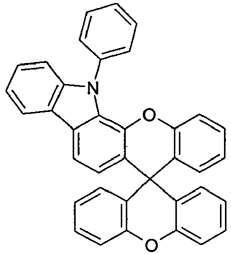 | 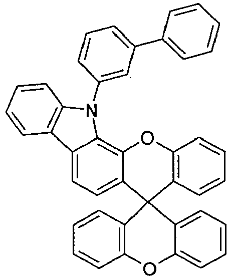 |
| 101 | 102 |
| 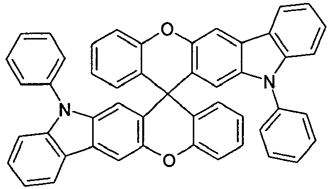 | 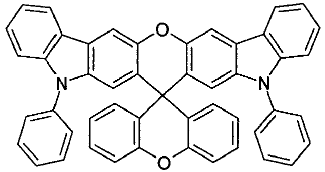 |
| 103 | 104 |
| 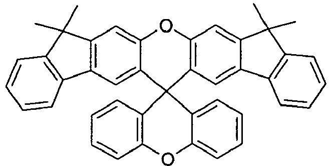 | 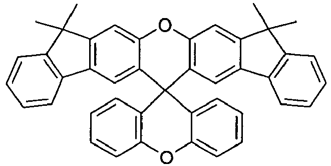 |
| 105 | 106 |
| 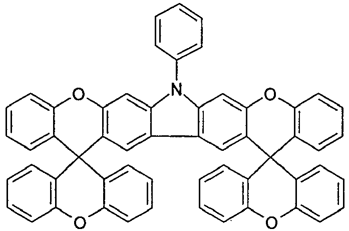 | |
| 107 | |
| 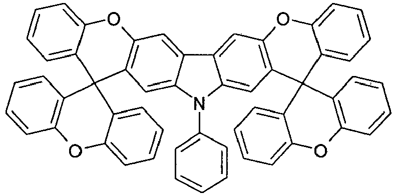 | |
| 108 | |
| 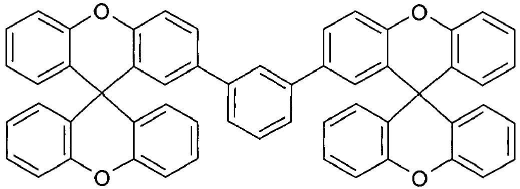 | |
| 109 | |

| | |
|---|---|
| * Verbindungen gemäß der Erfindung | |

Die erfindungsgemäßen Verbindungen können nach dem in Schema 1 skizzierten Weg dargestellt werden.

Die Metallierung eines 2-Halogen-substituierten Diarylethers (A), wobei das Halogen bevorzugt Brom ist, mit reaktiven Metallen (z. B. Magnesium nach Grignard) oder mit Organolithium-Verbindungen, gefolgt von Addition an mono-, di oder polyhalogeniertes Xanthon (B) und anschließende säurekatalysierte Cyclisierung des intermediären Alkoholats führt zu den entsprechenden halogensubstituierten Spiro-9,9-bixanthenen (C) (Schema 1). Dabei steht Hal für ein Halogen und R für einen Substituenten, wie er oben für R¹ definiert ist.

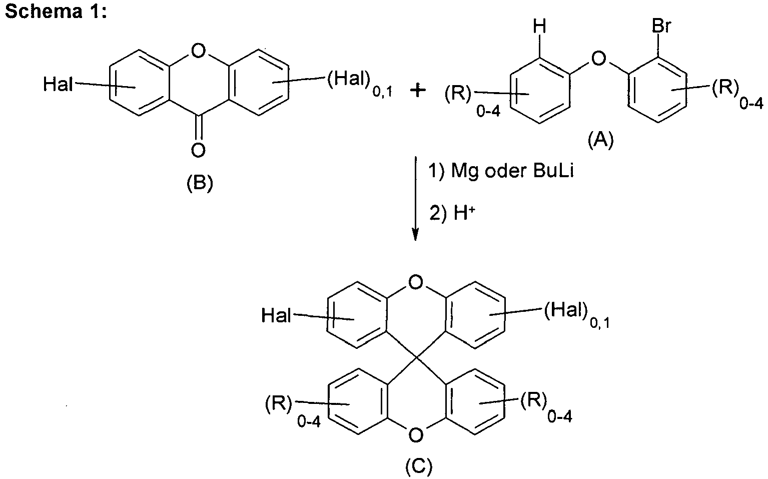

Die so entstandenen Halogenide, insbesondere Bromide, (C) können anschließend nach dem Fachmann geläufigen Methoden, wie zum Beispiel C-C-Kupplung, wie Suzuki-, Negishi-, Yamamoto-, Grignard-Cross-, Stille-, Heck-Kupplung, etc. oder C-N-Kupplung, wie Buchwald- oder Ullmann-Kupplung, Sililyierung, Phosphanylierung, Boranylierung, Poly-Kondensation, etc. weiter umgesetzt werden.

Ein weiterer Gegenstand der vorliegenden Erfindung ist ein Verfahren zur Herstellung einer Verbindung gemäß Formel (1), umfassend die Reaktionsschritte:
a) Synthese eines halogensubstituierten Spiro-9,9-bixanthens; und
b) Umsetzung des halogensubstituierten Spiro-9,9-bixanthens in einer C-C-Kupplung, wie Suzuki-, Negishi-, Yamamoto-, Grignard-Cross-, Stille-, Heck-Kupplung, etc., oder C-N-Kupplung, wie Buchwald- oder Ullmann-Kupplung, Sililyierung, Phosphanylierung, Boranylierung, Poly-Kondensation, etc..

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, oder mit reaktiven, polymerisierbaren Gruppen, wie Olefinen, Styrolen, Acrylaten oder Oxetanen, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität bzw. über die polymerisierbare Gruppe. Es ist weiterhin möglich, die Polymere über derartige Gruppen zu vernetzen. Die erfindungsgemäßen Verbindungen und Polymere können als vernetzte oder unvernetzte Schicht eingesetzt werden.

Weiterer Gegenstand der Erfindung sind daher Oligomere, Polymere oder Dendrimere enthaltend eine oder mehrere der oben aufgeführten erfindungsgemäßen Verbindungen, wobei an einer oder mehreren Positionen statt Substituenten eine oder mehrere Bindungen der erfindungsgemäßen Verbindung zum Polymer, Oligomer oder Dendrimer vorhanden sind. Je nach Verknüpfung der erfindungsgemäßen Verbindung bildet diese eine Seitenkette des Oligomers oder Polymers oder ist in der Hauptkette verknüpft oder bildet den Kern eines Dendrimers. Die Polymere, Oligomere oder Dendrimere können konjugiert, teilkonjugiert oder nicht-konjugiert sein. Die Oligomere oder Polymere können linear, verzweigt oder dendritisch sein. Für die Wiederholeinheiten der erfindungsgemäßen Verbindungen in Oligomeren, Dendrimeren und Polymeren gelten dieselben Bevorzugungen, wie oben beschrieben.

Zur Herstellung der Oligomere oder Polymere werden die erfindungsgemäßen Monomere homopolymerisiert oder mit weiteren Monomeren copolymerisiert. Bevorzugt sind Homopolymere oder Copolymere, wobei die Einheiten gemäß Formel (1) bzw. die oben ausgeführten bevorzugten Ausführungsformen zu 0.01 bis 99.9 mol%, bevorzugt 5 bis 90 mol%, besonders bevorzugt 20 bis 80 mol% vorhanden sind. Geeignete und bevorzugte Comonomere, welche das Polymergrundgerüst bilden, sind gewählt aus Fluorenen (z. B. gemäß EP 842208 oder WO 2000/22026), Spirobifluorenen (z. B. gemäß EP 707020, EP 894107 oder WO 2006/061181), Para-phenylenen (z. B. gemäß WO 92/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere können noch weitere Einheiten enthalten, beispielsweise Lochtransporteinheiten, insbesondere solche basierend auf Triarylaminen, und/oder Elektronentransporteinheiten. Außerdem können die Polymere entweder einpolymerisiert oder als Blend eingemischt TriplettEmitter enthalten. Gerade die Kombination von den erfindungsgemäßen Oligomere, Polymeren oder Dendrimeren mit Triplett-Emittern führt zu besonders guten Ergebnissen.

Die erfindungsgemäßen Verbindungen eignen sich für die Verwendung in einer elektronischen Vorrichtung, insbesondere in einer organischen Elektrolumineszenzvorrichtung.

Ein weiterer Gegenstand der vorliegenden Erfindung ist daher die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich bei der erfindungsgemäßen organischen Elektrolumineszenzvorrichtung auch um eine Tandem-OLED handeln, insbesondere auch für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen als Matrixmaterial für fluoreszierende oder phosphoreszierende Emitter und/oder als fluoreszierenden Emitter, insbesondere als blau fluoreszierenden Emitter, und/oder in einer elektronenblockierenden bzw. exzitonenblockierenden Schicht und/oder in einer Lochtransportschicht und/oder in einer Lochblockierschicht und/oder in einer Elektronentransportschicht, je nach genauer Substitution.

In einer weiteren Ausführungsform der Erfindung enthält die organische Elektrolumineszenzvorrichtung die erfindungsgemäße Verbindung in einer optischen Auskopplungsschicht. Unter einer optischen Auskopplungsschicht wird dabei eine Schicht verstanden, die nicht zwischen der Anode und der Kathode liegt, sondern die außerhalb der eigentlichen Vorrichtung auf eine Elektrode aufgebracht wird, beispielsweise zwischen einer Elektrode und einem Substrat, um die optische Auskopplung zu verbessern.

In einer bevorzugten Ausführungsform der Erfindung wird die erfindungsgemäße Verbindung als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die erfindungsgemäße Verbindung als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Besonders geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder der nicht offen gelegten Anmeldung EP 11007693.2 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und der nicht offen gelegten Anmeldung EP 11003232.3, oder Triphenylenderivate, z. B. gemäß der nicht offen gelegten Anmeldung DE 102010048608.6. Ebenso kann ein weiterer phosphoreszierender Emitter, welcher kürzerwellig als der eigentliche Emitter emittiert, als Co-Host in der Mischung vorhanden sein.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 2005/033244, WO 2005/019373, US 2005/0258742, WO 2010/086089, WO 2011/157339 und WO 2012/007086 entnommen werden. Weiterhin eignen sich beispielsweise die in den nicht offen gelegten Anmeldungen EP 11004545.7, EP 11005252.9 und EP 11006562.0 offenbarten Metallkomplexe. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren Ausführungsform der Erfindung wird die erfindungsgemäße Verbindung in einer Lochtransportschicht bzw. in einer Elektronenblockierschicht bzw. Exzitonenblockierschicht eingesetzt.

In nochmals einer weiteren bevorzugten Ausführungsform der Erfindung wird die erfindungsgemäße Verbindung als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. LiQ (Lithiumhydroxychinolinat).

In nochmals einer weiteren bevorzugten Ausführungsform der Erfindung wird die erfindungsgemäße Verbindung in einer Lochblockierschicht eingesetzt. Unter einer Lochblockierschicht wird eine Schicht verstanden, die auf Kathodenseite direkt an eine emittierende Schicht angrenzt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen bzw. Verbindungen gemäß Formel (1) bzw. die oben ausgeführten bevorzugten Ausführungsformen, eingesetzt als Matrixmaterial für fluoreszierende oder phosphoreszierende Emitter, führen zu hohen Effizienzen sowie zu langen Lebensdauern. Dies gilt insbesondere, wenn die Verbindungen als Matrixmaterial für einen phosphoreszierenden Emitter eingesetzt werden.
2. Die erfindungsgemäßen Verbindungen bzw. Verbindungen gemäß Formel (1) bzw. die oben ausgeführten bevorzugten Ausführungsformen eignen sich nicht nur als Matrix für rot phosphoreszierende Verbindungen, sondern auch für grün und gegebenenfalls auch für blau phosphoreszierende Verbindungen.
3. Die erfindungsgemäßen Verbindungen lassen sich in sehr hoher Ausbeute und sehr hoher Reinheit herstellen, wodurch eine aufwändige Reinigung, die immer auch mit Materialverlusten verbunden ist, entfallen kann oder zumindest nur in erheblich geringerem Ausmaß erforderlich ist.
4. Die erfindungsgemäßen Verbindungen weisen eine hohe thermische Stabilität auf, was nicht nur bei der Herstellung der OLEDs durch

Vakuumverdampfung, sondern auch bei der Aufreinigung durch Sublimationsverfahren Vorteile bietet.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### Beispiel 1: 2,7-Dibrom-sipro-9,9'-bixanthen, Synthon S1

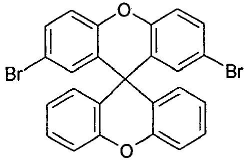

Aus 95.6 g (380 mmol) 2-Bromphenyl-phenylether [7025-06-1] in einem Gemisch aus 2.6 ml (34 mmol) 1,2-Dichlorethan und 1000 ml THF wird mit 10.8 g (410 mmol) mit Iod aktivierten Magnesiumspänen das entsprechende Grignard-Reagenz dargstellt. Nachdem das Magnesium vollständig abreagiert hat, trägt man in die Lösung portionsweise 112.0 g (316 mmol) 2,7-Dibromxanthon [40102-85-0] fest ein und rührt die Reaktionsmischung weitere 6 h unter Rückfluss nach. Man destilliert 500 ml THF ab, lässt die Suspension unter Rühren auf 30 °C erkalten, tropft 900 ml Eisessig zügig zu (ACHTUNG: exotherm!), versetzt die rote Lösung tropfenweise mit einem Gemisch aus 100 ml Eisessig und 30 ml konz. Schwefelsäure, rührt 6 h bei 60 °C nach, lässt auf 30 °C erkalten, tropft 600 ml Ethanol und dann 400 ml eines Gemischs aus Ethanol: Wasser (1:1 v:v) zu, rührt 1 h nach, saugt vom farblosen Feststoff ab, wäscht diesen zweimal mit je 150 ml Eisessig, zweimal mit je 200 ml eines Gemischs aus Ethanol : Wasser (1:1 v:v) und trocknet diesen im Vakuum. Mann nimmt den Feststoff in 2000 ml Dichlormethan auf, saugt von unlöslichen Anteilen über ein kurzes Celite-Bett ab, entfernt das Dichlormethan im Vakuum und kristallisiert dann einmal aus DMF um. Ausbeute: 121.6 g (240 mmol), 76 %; Reinheit: ca. 99.5 % ig n. ¹H-NMR.

Analog werden folgende Verbindungen erhalten:

| Bsp. | Bromdiarylether | Xanthon | Produkt | Ausbeute |
|---|---|---|---|---|
| 2 | 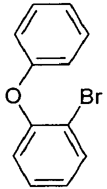 | 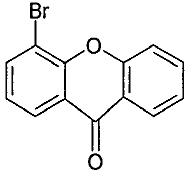 | 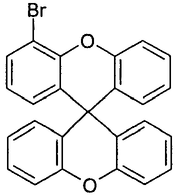 | 73% |
| | | 861548-92-7 | S2 | |
| 3 | 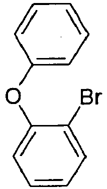 | 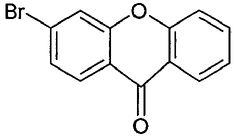 | 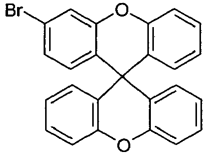 | 78% |
| | | 500286-36-2 | S3 | |
| 4 | 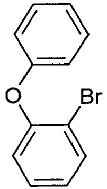 | 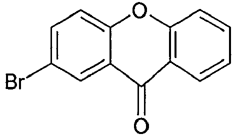 | 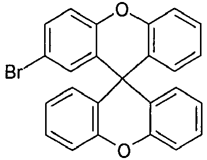 | 77% |
| | | 56341-31-2 | S4 | |
| 5 | 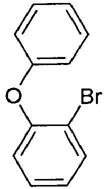 | 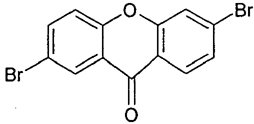 | 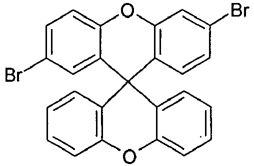 | 68 % |
| | | 1246661-43-7 | S5 | |
| 6 | 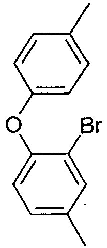 | 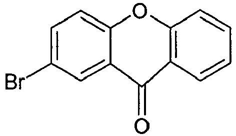 | 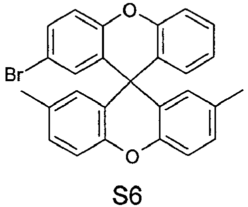 | 70% |
| | 27044-93-5 | | | |
| 7 | 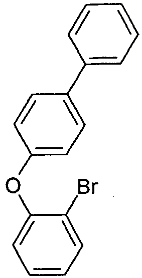 | 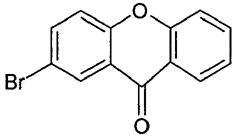 | 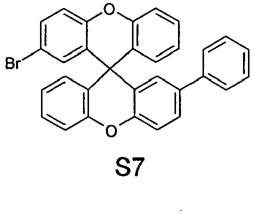 | 66% |
| | 556113-49-6 | | | |

### Beispiel 8: 2,7-Bis(diphenylamino)-sipro-9,9'-spiro-bixanthen

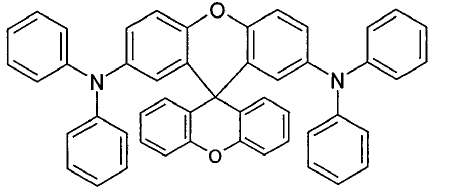

Ein Gemisch aus 50.6 g (100 mmol) 2,7-Dibrom-spiro-9,9'-spiro-bixanthen S1, 37.2 g (220 mmol) Diphenylamin, 25.0 g (260 mmol) Natrium-tert-butylat, 809 mg (4 mmol) Tri-tert-butylphosphin, 449 mg (2 mmol) Palladium(II)acetat und 1000 ml Toluol wird 16 h unter Rückfluss erhitzt. Man lässt die Reaktionsmischung auf 50 °C erkalten, gibt 500 ml Wasser zu, trennt die organische Phase ab, wäscht diese zweimal mit 500 ml Wasser, trocknet über Magnesiumsulfat, filtriert über ein Celite-Bett (5 cm) ab und engt dann im Vakuum zur Trockene ein. Das Rohprodukt wird zweimal aus DMF und dreimal aus Dioxan umkristallisiert und abschließend zweimal fraktioniert sublimiert (p ca. 10⁻⁵ mbar, T = 330 °C). Ausbeute: 32.9 g (48 mmol) 48 %, Reinheit: 99.9 % n. HPLC.

Analog werden folgende Verbindungen erhalten, wobei bei Monobromiden 200 mmol eingesetzt werden:

| Bsp. | Amin | Bromid | Produkt | Ausbeute |
|---|---|---|---|---|
| 9 | 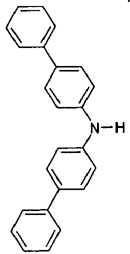 | 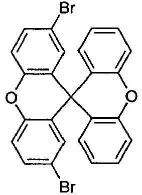 | 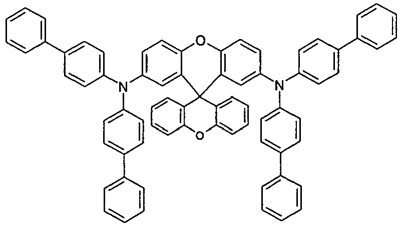 | 45% |
| | 102113-98-4 | S1 | | |
| 10 | 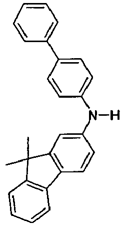 | 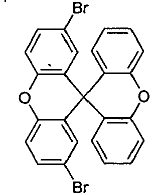 | 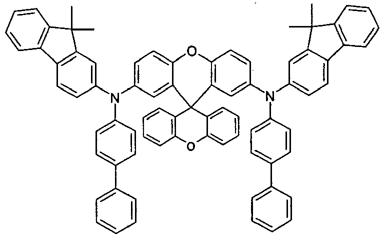 | 51 % |
| | 897671-69-1 | S1 | | |
| 11 | 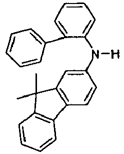 | 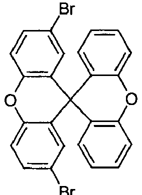 | 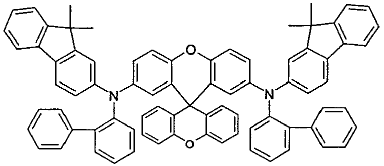 | 44% |
| | 1198395-24-2 | S1 | | |
| 12 | 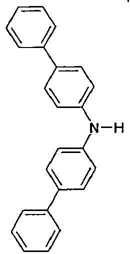 | 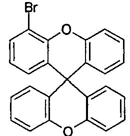 | 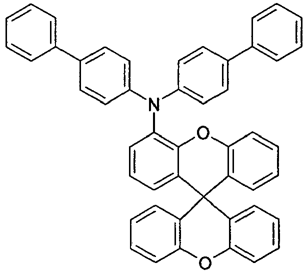 | 50% |
| | 102113-98-4 | S2 | | |
| 13 | 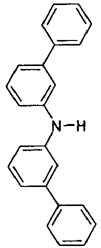 | 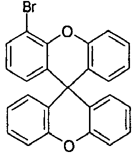 | 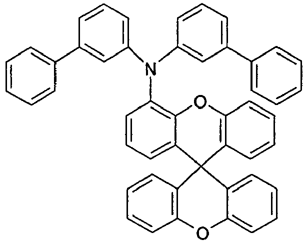 | 46% |
| | 169224-65-1 | S2 | | |
| 14 | 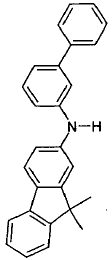 | 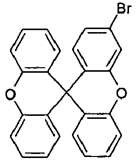 | 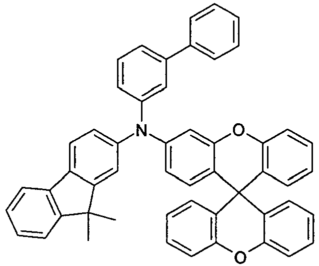 | 45% |
| | | S3 | | |
| 15 | 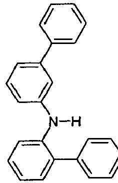 | 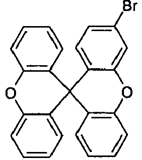 | 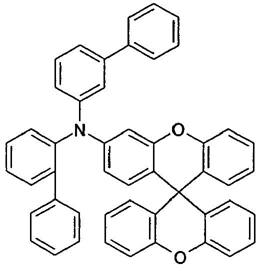 | 46% |
| | | S3 | | |
| 16 | 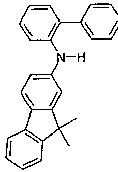 > | 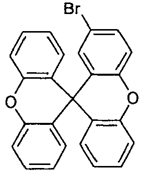 | 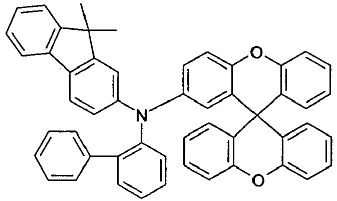 | 41 % |
| | 1198395-24-2 | S4 | | |
| 17 | 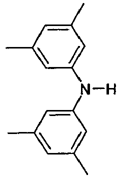 | 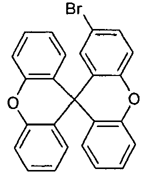 | 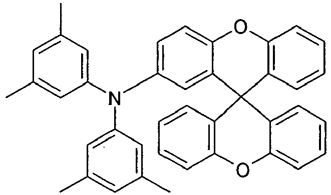 | 49% |
| | 5369-25-5 | S4 | | |
| 18 | 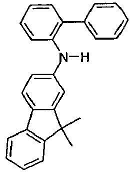 | 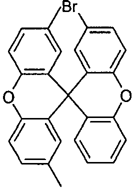 | 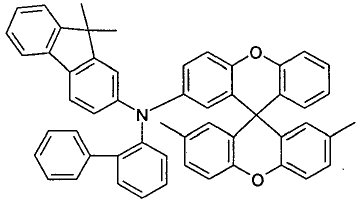 | 42% |
| | 1198395-24-2 | S5 | | |
| 19 | 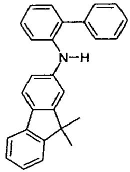 | 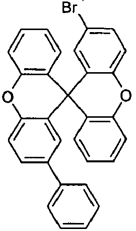 | 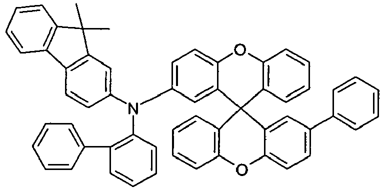 | 46% |
| | 1198395-24-2 | S6 | | |
| 20 | 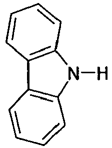 | 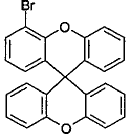 | 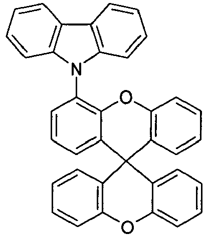 | 50% |
| | 86-74-8 | S2 | Anstelle von Natrium-tert-butylat wird Kaliumcarbonat, anstelle von Toluol wird o-Xylol verwendet. | |
| 21 | 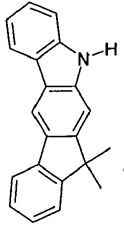 | 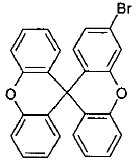 | 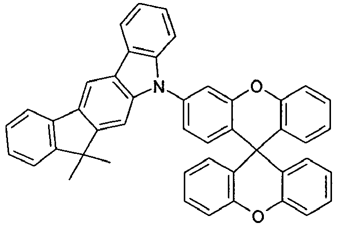 | 38 % |
| | 1257220-47-5 | S3 | Anstelle von Natrium-tert-butylat wird Kaliumcarbonat, anstelle von Toluol wird o-Xylol verwendet | |

### Beispiel 22: 2,7-Dibrom-sipro-9,9'-spiro-bixanthen

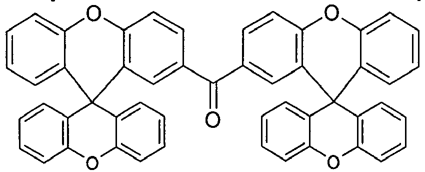

Eine auf - 78 °C gekühlte Lösung von 42.7 g (100 mmol) 2-Brom-spiro-9,9'-spiro-bixanthen S4 in 1500 ml THF wird tropfenweise mit 40 ml (100 mmol) n-Butyllithium (2.5M in n-Hexan) versetzt und 30 min. nachgerührt. Dann gibt man 4.6 ml (50 mmol) N,N-Dimethylcarbamoylchlorid [79-44-7], verdünnt mit 10 ml THF, auf ein Mal zu, rührt 30 min. nach, lässt die Reaktionsmischung auf 0 °C erwärmen, versetzt mit 50 ml konz. Salzsäure und erhitzt die Reaktionsmischung 5 h unter Rückfluss. Nach Erkalten entfernt man das THF im Vakuum, nimmt den Rückstand in 200 ml Ethanol auf, stellt mit 10 % iger Ammoniaklösung schwach alkalisch, saugt vom ausgefallenen Festsoff ab, wäscht diesen dreimal mit je 100 ml eines Ethanol-Wasser-Gemischs (1:1 vv) und abschließend einmal mit 50 ml Ethanol. Das Rohprodukt wird fünfmal aus DMF umkristallisiert und abschließend zweimal fraktioniert sublimiert (p ca. 10⁻⁵ mbar, T = 340 °C). Ausbeute: 19.5 g (27 mmol) 54 %, Reinheit: 99.9 % n. HPLC.

Analog werden folgende Verbindungen erhalten:

| Bsp. | Elektrophil | Bromid | Produkt | Ausbeute |
|---|---|---|---|---|
| 23 | 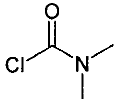 | 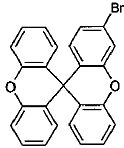 | 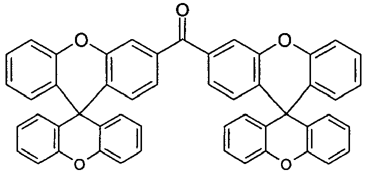 | 57% |
| | | S3 | | |
| 24 | 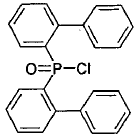 | 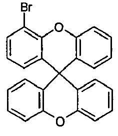 | 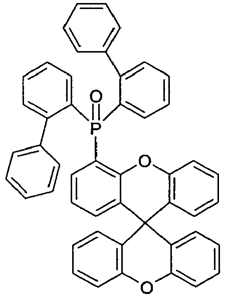 | 42% |
| | 1351669-38-9 100 mmol Elektrophil | S2 | Der Hydrolyseschritt mit konz. HCl entfällt. | |
| 25 | 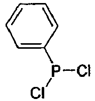 | 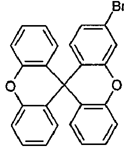 | 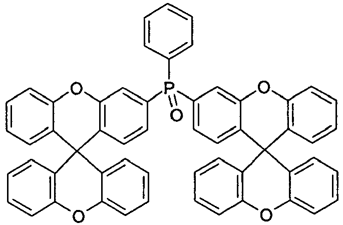 | 58 % |
| | 1499-21-4 | S3 | Der Hydrolyseschritt mit konz. HCl entfällt. | |
| 26 | 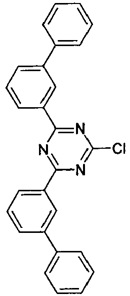 | 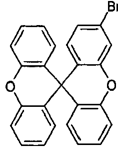 | 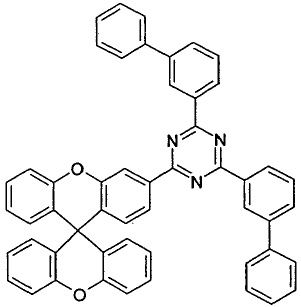 | 53% |
| | 1205748-61-3 100 mmol Elektrophil | S3 | Der Hydrolyseschritt mit konz. HCl entfällt. | |

### Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen O1 bis 022 (siehe Tabellen 1 bis 5) werden der Aufbau sowie die Daten verschiedener OLEDs vorgestellt. Glasplättchen, die mit strukturiertem ITO (Indium Zinn Oxid) der Dicke 150 nm beschichtet sind, werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf welche die OLEDs aufgebracht werden. Die OLEDs haben prinzipiell folgenden Schichtaufbau: Substrat / optionale Lochinjektionsschicht (HIL) / Lochtransportschichten (HTL) / Zwischenschicht (IL) / Elektronenblockerschicht (EBL) / Emissionsschicht (EML) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist den Tabellen 1 und 3 zu entnehmen. Die zur Herstellung der OLEDs benötigten Materialien sind in Tabelle 5 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB1 (95%:5%) bedeutet hierbei, dass das Material H1 in einem Volumenanteil von 95% und SEB1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U @ 1000 cd/m² in Tabelle 2 und 4 bezeichnet die Spannung, die für eine Leuchtdichte von 1000 cd/m² benötigt wird. EQE @ 1000 cd/m² schließlich bezeichnet die externe Quanteneffizienz bei einer Betriebsleuchtdichte von 1000 cd/m². LD80 @ 6000 cd/m2 ist die Lebensdauer bis das OLED bei einer Helligkeit von 6000 cd/m² auf 80 % der Anfangsintensität, also auf 4800 cd/m² abgefallen ist.

Die gemessenen Daten der verschiedenen OLEDs sind in den Tabellen 2 und 4 zusammengefasst.

### Verwendung der erfindungsgemäßen Verbindungen in fluoreszierenden und phosphoreszierenden OLEDs

Die erfindungsgemäßen Verbindungen eignen sich insbesondere als HTM (Lochtransportmaterial) oder EBM (Elektronenblockiermaterial) in OLEDs. Sie eignen sich zur Verwendung in einer Einzelschicht, aber auch als Komponente einer Mischung als HTM, EBM oder als Bestandteil der emittierenden Schicht. Verglichen mit Vergleichs-Vorrichtungen gemäß dem Stand der Technik (V1 und V2) zeigen alle Proben mit den erfindungsgemäßen Verbindungen höhere Effizienzen und/oder verbesserte Lebensdauern. Im Vergleich zum Referenzmaterial NPB zeigen die erfindungsgemäßen Verbindungen bessere Effizienzen und bessere Lebensdauern.

| **Tabelle 1: Aufbau der OLEDs** | | | | | | | |
|---|---|---|---|---|---|---|---|
| **Bsp.** | **IL** | **HTL** | **IL** | **HTL2** | **EBL** | **EML** | **ETL** |
| | Dicke / nm | Dicke / nm | Dicke / nm | Dicke / nm | Dicke / nm | Dicke / nm | Dicke / nm |
| V1 | HIL1 5 nm | HIL2 130 nm | HIL1 5nm | NPB 10 nm | NPB 20 nm | H1(95%):SEB1(5%) 20 nm | ETM1(50%):LiQ(50%) 30 nm |
| O1 | HIL1 5 nm | HIL2 130 nm | HIL1 5 nm | NPB 10 nm | Bsp.8 20 nm | H1(95%):SEB1(5%) 20 nm | ETM1(50%):LiQ(50%) 30 nm |
| O2 | HIL1 5 nm | HIL2 130 nm | HIL1 5nm | - | Bsp. 9 30 nm | H1(95%):SEB1(5%) 20 nm | ETM1(50%):LiQ(50%) 30 nm |
| O3 | HIL1 5nm | HIL2 130 nm | HIL1 5nm | - | Bsp.10 20 nm | H1(95%):SEB1(5%) 20 nm | ETM1(50%):LiQ(50%) 30 nm |
| O4 | HIL1 5nm | HIL2 130 nm | HIL1 5nm | - | Bsp.11 30 nm | H1(95%):SEB1(5%) 20 nm | ETM1(50%):LiQ(50%) 30 nm |
| O5 | HIL1 5 nm | HIL2 130 nm | HIL1 5nm | - | Bsp.12 30 nm | H1(95%):SEB1(5%) 20 nm | ETM1(50%):LiQ(50%) 30 nm |
| O6 | HIL1 5nm | HIL2 130 nm | HIL1 5nm | - | Bsp.13 30 nm | H1(95%):SEB1(5%) 20 nm | ETM1(50%):LiQ(50%) 30 nm |
| O7 | HIL1 5 nm | HIL2 130 nm | HIL1 5nm | - | Bsp.14 30 nm | H1(95%):SEB1(5%) 20 nm | ETM1(50%):LiQ(50%) 30 nm |
| O8 | HIL1 5 nm | HIL2 130 nm | HIL1 5nm | - | Bsp.15 30 nm | H1(95%):SEB1(5%) 20 nm | ETM1(50%):LiQ(50%) 30 nm |
| O9 | HIL1 5nm | HIL2 130 nm | HIL1 5nm | - | Bsp.16 30 nm | H1(95%):SEB1(5%) 20 nm | ETM1(50%):LiQ(50%) 30 nm |
| O10 | HIL1 5 nm | HIL2 130 nm | HIL1 5nm | - | Bsp.17 30 nm | H1(95%):SEB1(5%) 20 nm | ETM1(50%):LiQ(50%) 30 nm |
| O11 | HIL1 5 nm | HIL2 130 nm | HIL1 5nm | - | Bsp.18 30 nm | H1(95%):SEB1(5%) 20 nm | ETM1(50%):LiQ(50%) 30 nm |
| O12 | HIL1 5 nm | HIL2 130 nm | HIL1 5nm | - | Bsp.19 30 nm | H1(95%):SEB1(5%) 20 nm | ETM1(50%):LiQ(50%) 30 nm |

| **Tabelle 2: Daten der OLEDs** | | | | | |
|---|---|---|---|---|---|
| **Bsp.** | **U @ 1000 cd/m2** | **EQE @ 1000 cd/m2** | **LD80 @ 6000 cd/m²** | **CIE** | |
| | V | % | [h] | x | y |
| V1 | 4.7 | 4.8 | 70 | 0.14 | 0.17 |
| O1 | 4.3 | 6.7 | 80 | 0.14 | 0.16 |
| O2 | 4.3 | 6.6 | 100 | 0.14 | 0.16 |
| O3 | 4.4 | 6.4 | 95 | 0.14 | 0.16 |
| O4 | 4.4 | 7.2 | 105 | 0.14 | 0.16 |
| O5 | 4.4 | 6.9 | 120 | 0.14 | 0.16 |
| O6 | 4.4 | 7.0 | 110 | 0.14 | 0.16 |
| O7 | 4.5 | 7.0 | 120 | 0.14 | 0.16 |
| O8 | 4.4 | 7.1 | 125 | 0.14 | 0.16 |
| O9 | 4.6 | 7.3 | 130 | 0.14 | 0.16 |
| O10 | 4.6 | 6.7 | 85 | 0.14 | 0.16 |
| O11 | 4.3 | 7.1 | 115 | 0.14 | 0.16 |
| O12 | 4.5 | 7.0 | 120 | 0.14 | 0.16 |

| **Tabelle 3: Aufbau der OLEDs** | | | | | | |
|---|---|---|---|---|---|---|
| **Bsp.** | **HTL** | **IL** | **HTL2** | **EBL** | **EML** | **ETL** |
| | Dicke / nm | Dicke / nm | Dicke / nm | Dicke / nm | Dicke / nm | Dicke / nm |
| V2 | HIL2 70 nm | HIL1 5 nm | - | NPB 90 nm | H2(88%):Irpy(12%) 30 nm | ETM1(50%):LiQ(50%) 40 nm |
| O13 | HIL2 70 nm | HIL1 5 nm | - | Bsp.14 80 nm | H2(88%):Irpy(12%) 30 nm | ETM1(50%):LiQ(50%) 40 nm |
| O14 | HIL2 70 nm | HIL1 5 nm | - | Bsp. 15 80 nm | H2(88%):Irpy(12%) 30 nm | ETM1(50%):LiQ(50%) 40 nm |
| O15 | HIL2 70 nm | HIL1 5 nm | - | Bsp.15 90 nm | H3(30%): Bsp.22(65%) :Irpy(5%) 30 nm | ETM1(50%):LiQ(50%) 40 nm |
| O16 | HIL2 70 nm | HIL1 5nm | - | Bsp.15 90 nm | H3(20%): Bsp.23(75%) :Irpy(5%) 30 nm | ETM1(50%):LiQ(50%) 40 nm |
| O17 | HIL2 70 nm | HIL1 5 nm | - | Bsp.15 90 nm | H3(30%): Bsp.24(65%) :Irpy(5%) 30 nm | ETM1(50%):LiQ(50%) 40 nm |
| O18 | HIL2 70 nm | HIL1 5 nm | - | Bsp.15 90 nm | H3(30%): Bsp.25(65%) :Irpy(5%) 30 nm | ETM1(50%):LiQ(50%) 40 nm |
| O19 | HIL2 70 nm | HIL1 5 nm | - | Bsp.15 90 nm | H3(25%): Bsp.26(70%) :Irpy(5%) 30 nm | ETM1(50%):LiQ(50%) 40 nm |
| O20 | HIL2 70 nm | HIL1 5 nm | - | Bsp.15 90 nm | Bsp.15(30%): Bsp.26(65%):Irpy(5%) 30 nm | ETM1(50%):LiQ(50%) 40 nm |
| O21 | HIL2 70 nm | HIL1 5 nm | - | Bsp.14 80 nm | Bsp.20(88%):Irpy(12%) 30 nm | ETM1(50%):LiQ(50%) 40 nm |
| O22 | HIL2 70 nm | HIL1 5 nm | - | Bsp.14 80 nm | Bsp.21(88%):Irpy(12%) 30 nm | ETM1(50%):LiQ(50%) 40 nm |

| **Tabelle 4: Daten der OLEDs** | | | | | |
|---|---|---|---|---|---|
| **Bsp.** | **U @ 1000 cd/m2** | **EQE @ 1000 cd/m2** | **LD80 @ 8000 cd/m²** | **CIE** | |
| | V | % | [h] | x | y |
| V2 | 3.5 | 14.4 | 90 | 0.32 | 0.63 |
| O13 | 3.3 | 18.5 | 120 | 0.33 | 0.63 |
| O14 | 3.3 | 18.7 | 130 | 0.33 | 0.63 |
| O15 | 3.2 | 18.9 | 140 | 0.33 | 0.64 |
| O16 | 3.3 | 19.0 | 150 | 0.33 | 0.63 |
| O17 | 3.3 | 18.4 | 110 | 0.33 | 0.64 |
| O18 | 3.6 | 18.7 | 105 | 0.33 | 0.63 |
| O19 | 3.4 | 18.7 | 140 | 0.33 | 0.63 |
| O20 | 3.2 | 18.8 | 135 | 0.33 | 0.64 |
| O21 | 3.4 | 18.0 | 90 | 0.33 | 0.64 |
| O22 | 3.4 | 18.3 | 125 | 0.33 | 0.64 |

**Tabelle 5:**

| | | |
|---|---|---|
| 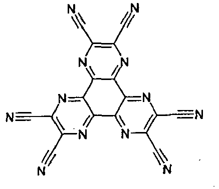 | 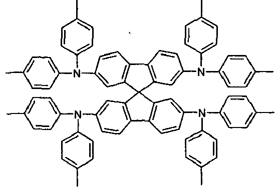 | 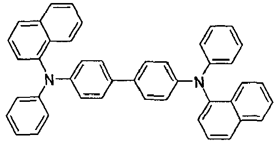 |
| HIL1 | HIL2 | NPB |
| 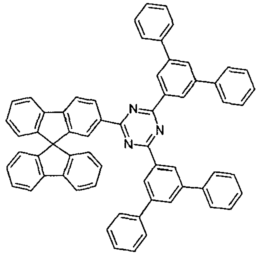 | 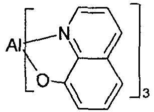 | 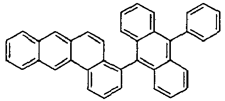 |
| ETM1 | Alq3 | H1 |
| 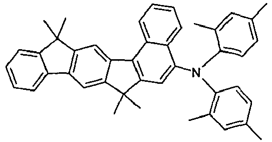 | 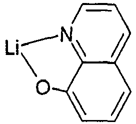 | 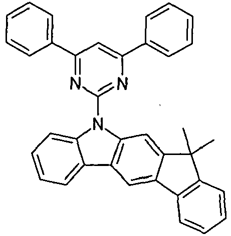 |
| SEB1 | LiQ | H2 |
| 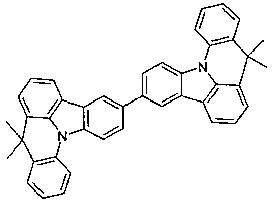 | 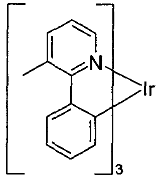 | |
| H3 | Irpy | |

## Patentansprüche

1. Verbindung gemäß einer von den Formeln (5a) bis (5u), 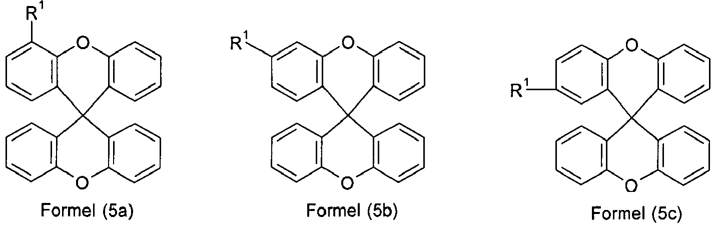 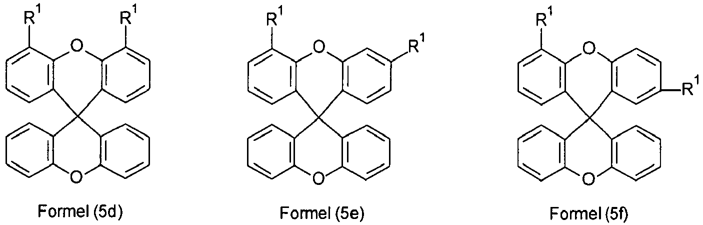 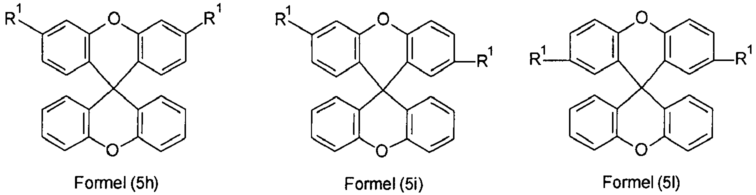 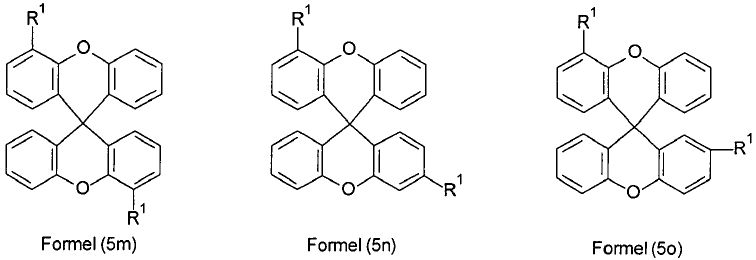 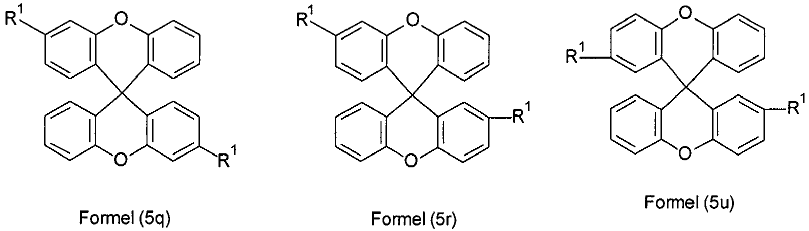 und wobei für die verwendeten Symbole und Indizes gilt:
R¹ ist bei jedem Auftreten gleich oder verschieden ausgewählt aus der Gruppe bestehend aus H, D, F, Cl, Br, I, CN, NO₂, N(Ar¹)₂, N(R³)₂, C(=O)Ar¹, C(=O)R³, P(=O)(Ar¹)₂, B(Ar¹)₂, Si(Ar¹)₃, Si(R³)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R³ substituiert sein kann, wobei eine oder mehrere nicht-benachbarte CH₂-Gruppen durch R³C=CR³, C=C, Si(R³)₂, C=O, C=S, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R³ substituiert sein kann, einer Aryloxy- oder Heteroaryloxygruppe mit 5 bis 60 aromatischen Ringatomen, die mit einem oder mehreren Resten R³ substituiert sein kann.
Ar¹ ist bei jedem Auftreten gleich oder verschieden ein aromatisches oder heteroaromatisches Ringsystem mit 5-30 aromatischen Ringatomen, das mit einem oder mehreren nicht-aromatischen Resten R³ substituiert sein kann; dabei können zwei Reste Ar¹, welche an dasselbe N-Atom oder P-Atom binden, auch durch eine Einfachbindung oder eine Brücke, ausgewählt aus N(R³), C(R³)₂, O oder S, miteinander verbrückt sein;
R³ ist bei jedem Auftreten gleich oder verschieden ausgewählt aus der Gruppe bestehend aus H, D, F, Cl, Br, I, CN, NO₂, N(R⁴)₂, C(=O)R⁴, P(=O)(R⁴)₂, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, die jeweils mit einem oder mehreren Resten R⁴ substituiert sein kann, wobei eine oder mehrere nicht-benachbarte CH₂-Gruppen durch R⁴C=CR⁴, C≡C, Si(R⁴)₂, C=O, C=S, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 60 aromatischen Ringatomen, das jeweils mit einem oder mehreren Resten R⁴ substituiert sein kann, einer Aryloxy- oder Heteroaryloxygruppe mit 5 bis 60 aromatischen Ringatomen, die mit einem oder mehreren Resten R⁴ substituiert sein kann, oder einer Kombination dieser Systeme, wobei optional zwei oder mehr benachbarte Substituenten R³ ein monocyclisches oder polycyclisches, aliphatisches Ringsystem bilden können, das mit einem oder mehreren Resten R⁴ substituiert sein kann;
R⁴ ist bei jedem Auftreten gleich oder verschieden ausgewählt aus der Gruppe bestehend aus H, D, F, CN, einem aliphatischen Kohlenwasserstoffrest mit 1 bis 20 C-Atomen, einem aromatischen oder heteroaromatischen Ringsystem mit 5 bis 30 aromatischen Ringatomen, in dem ein oder mehrere H-Atome durch D, F, Cl, Br, I oder CN ersetzt sein können, wobei zwei oder mehr benachbarte Substituenten R⁴ miteinander ein mono- oder polycyclisches, aliphatisches Ringsystem bilden können;
mit der Maßgabe, dass in den Formeln (5a) bis (5u) mindestens ein Substituent R¹ vorhanden ist, der ausgewählt ist aus der Gruppe bestehend aus CN, N(Ar¹)₂, C(=O)Ar¹, P(=O)(Ar¹)₂, B(Ar¹)₂, Si(Ar¹)₃, Si(R³)₃ oder einem aromatischen oder heteroaromatischen Ringsystem mit 5 bis 60 aromatischen Ringatomen, das jeweils mit einem oder mehreren Resten R³ substituiert sein kann.

2. Verbindung nach Anspruch 1, wobei die Verbindung aus den Formeln (5d) bis (5u) ausgewählt ist, wobei eine Gruppe R¹ eine lochtransportierende Einheit und die andere Gruppe R¹ eine elektronentransportierende Einheit ist.

3. Verbindung nach Anspruch 1 oder 2, **dadurch gekennzeichnet, dass**, wenn R¹ für ein aromatisches oder heteroaromatisches Ringsystem steht, dieses gleich oder verschieden bei jedem Auftreten ausgewählt ist aus Benzol, ortho-, meta- oder para-Biphenyl, ortho-, meta-, para- oder verzweigtes Terphenyl, ortho-, meta- para- oder verzweigtes Quaterphenyl, 1- oder 2-Naphthyl, Pyrrol, Furan, Thiophen, Indol, Benzofuran, Benzothiophen, Carbazol, Dibenzofuran, Dibenzothiophen, Pyridin, Pyrimidin, Pyrazin, Pyridazin, Triazin, Anthracen, Phenanthren, Pyren, Benzanthracen oder Kombinationen aus zwei oder drei dieser Gruppen, welche jeweils mit einem oder mehreren Resten R³ substituiert sein können.

4. Verbindung nach einem oder mehreren der Ansprüche 1 bis 3, **dadurch gekennzeichnet, dass** die Verbindung, wenn sie als Elektronentransportmaterial oder als Matrixmaterial für einen phosphoreszierenden Emitter verwendet wird, mit mindestens einer Gruppe -C(=O)Ar¹ oder -P(=O)(Ar¹)₂ oder einem elektronenarmen heteroaromatischen Ringsystem substituiert ist, wobei bevorzugt mindestens ein Substituent R¹ ausgewählt ist aus Strukturen gemäß Formel (13) bis (16), 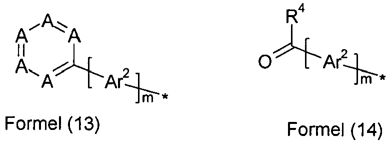 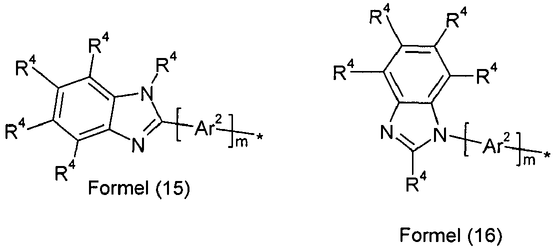 wobei R⁴ die in Anspruch 1 genannte Bedeutung hat, * die Position der Bindung der Gruppe gemäß Formel (13) bis (16) andeutet und weiterhin gilt:
m ist 0 oder 1;
A ist bei jedem Auftreten gleich oder verschieden CR⁴ oder N, mit der Maßgabe, dass eine, zwei oder drei Gruppen A für N stehen;
Ar² ist gleich oder verschieden bei jedem Auftreten ein bivalentes aromatisches oder heteroaromatisches Ringsystem mit 5 bis 16 C-Atomen, welches durch einen oder mehrere Reste R⁴ substituiert sein kann.

5. Verbindung nach einem oder mehreren der Ansprüche 1 bis 4, **dadurch gekennzeichnet, dass** die Verbindung, wenn sie als Lochtransportmaterial, als Lochinjektionsmaterial oder als Elektronen- bzw. Exzitonenblockiermaterial oder als Matrixmaterial für einen phosphoreszierenden Emitter verwendet wird, mit mindestens einem Triarylaminderivat, Carbazolderivat, Indenocarbazolderivat, Indolocarbazolderivat, Azacarbazolderivat, Indolderivat, Furanderivat, Benzofuranderivat, Dibenzofuranderivat, Thiophenderivat, Benzothiophenderivat oder Dibenzothiophenderivat, welche jeweils durch einen oder mehrere Reste R³ substituiert sein können, substituiert ist oder mindestens ein Substituent R¹ für -N(Ar¹)₂ steht, wobei diese Gruppen bevorzugt ausgewählt sind aus den Gruppen der folgenden Formeln (35) bis (49), 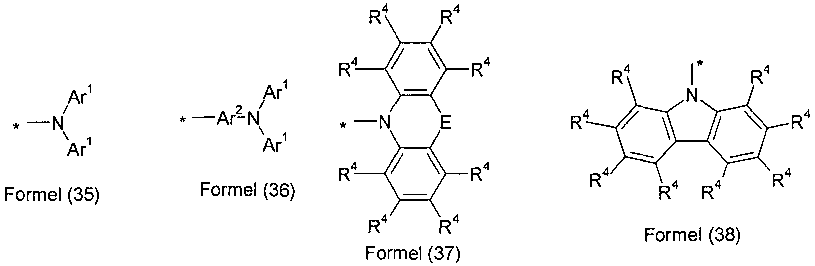 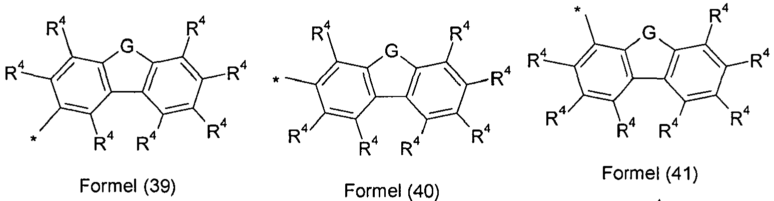 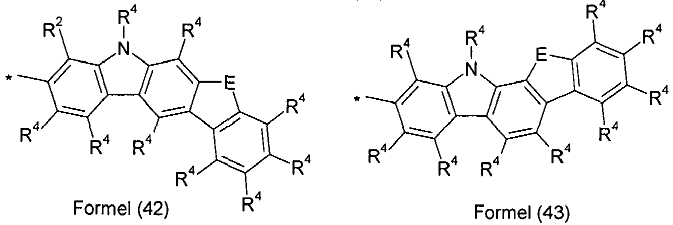 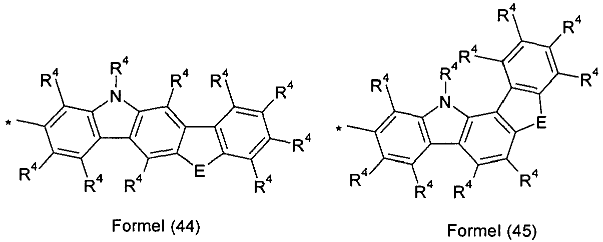 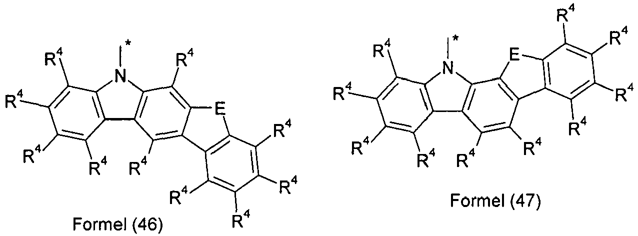 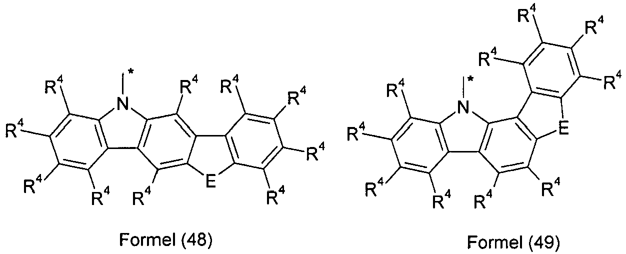 wobei die verwendeten Symbole die in Anspruch 1 und 4 genannten Bedeutungen aufweisen und weiterhin gilt:
E ist ausgewählt aus der Gruppe bestehend aus C(R³)₂, NR³, O oder S;
G ist ausgewählt aus der Gruppe bestehend aus NR³, O oder S.

6. Verfahren zur Herstellung einer Verbindung nach einem oder mehreren der Ansprüche 1 bis 5, umfassend die Reaktionsschritte:
a) Synthese eines halogensubstituierten Spiro-9,9-bixanthens; und
b) Umsetzung des halogensubstituierten Spiro-9,9-bixanthens in einer C-C-Kupplung, C-N-Kupplung, Sililyierung, Phosphanylierung, Boranylierung oder Poly-Kondensation.

7. Oligomer, Polymer oder Dendrimer enthaltend eine oder mehrere Verbindungen nach einem oder mehreren der Ansprüche 1 bis 5, wobei an einer oder mehreren Positionen statt Substituenten eine oder mehrere Bindungen der Verbindung zum Polymer, Oligomer oder Dendrimer vorhanden sind.

8. Verwendung einer Verbindung nach einem oder mehreren der Ansprüche 1 bis 5 oder eines Oligomers, Polymers oder Dendrimers gemäß Anspruch 7 in einer elektronischen Vorrichtung, insbesondere in einer organischen Elektrolumineszenzvorrichtung.

9. Elektronische Vorrichtung enthaltend mindestens eine Verbindung nach einem oder mehreren der Ansprüche 1 bis 5 oder ein Oligomer, Polymer oder Dendrimer gemäß Anspruch 7, wobei die elektronische Vorrichtung insbesondere ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und "organic plasmon emitting devices".

10. Elektronische Vorrichtung nach Anspruch 9, wobei es sich um eine organische Elektrolumineszenzvorrichtung handelt, **dadurch gekennzeichnet, dass** die Verbindung nach einem oder mehreren der Ansprüche 1 bis 5 als Matrixmaterial für fluoreszierende oder phosphoreszierende Emitter und/oder als fluoreszierenden Emitter und/ oder in einer elektronenblockierenden bzw. exzitonenblockierenden Schicht und/oder in einer Lochtransportschicht und/oder in einer Lochinjektionsschicht und/oder in einer Lochblockierschicht und/oder in einer Elektronentransportschicht, je nach genauer Substitution.

## Claims

1. Compound of one of the formulae (5a) to (5u), 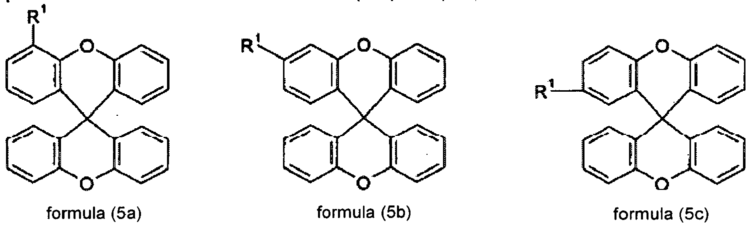 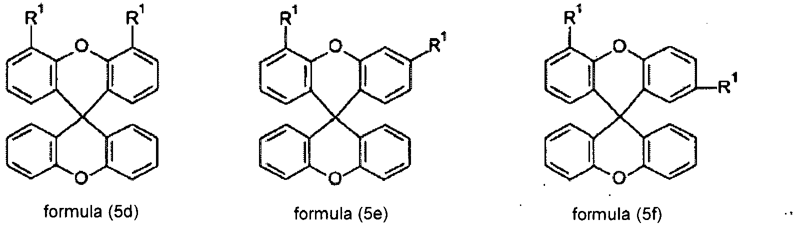 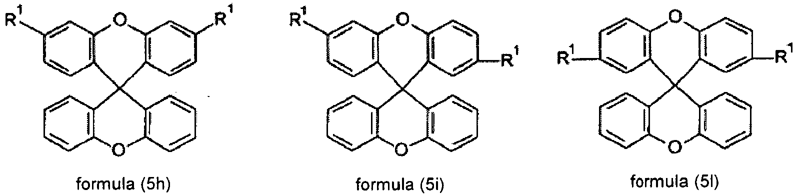 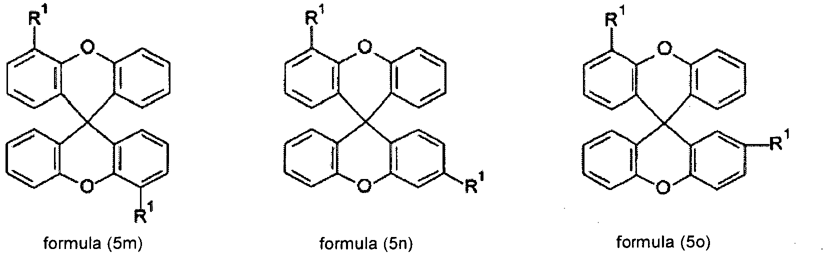 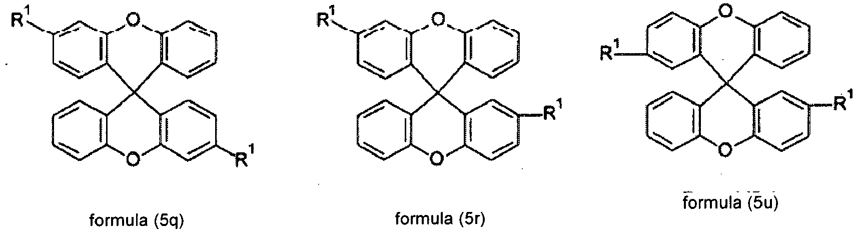 and where the following applies to the symbols and indices used:
R¹ is selected on each occurrence, identically or differently, from the group consisting of H, D, F, Cl, Br, I, CN, NO₂, N(Ar¹)₂, N(R³)₂, C(=O)Ar¹, C(=O)R³, P(=O)(Ar¹)₂, B(Ar¹)₂, Si(Ar¹)₃, Si(R³)₃, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R³, where one or more non-adjacent CH₂ groups may be replaced by R³C=CR³, C≡C, Si(R³)₂, C=O, C=S, C=NR³, P(=O)(R³), SO, SO₂, NR³, O, S or CONR³ and where one or more H atoms may be replaced by D, F, Cl, Br, I, CN or NO₂, an aromatic or heteroaromatic ring system having 5 to 60 aromatic ring atoms, which may in each case be substituted by one or more radicals R³, an aryloxy or heteroaryloxy group having 5 to 60 aromatic ring atoms, which may be substituted by one or more radicals R³;
Ar¹ is on each occurrence, identically or differently, an aromatic or heteroaromatic ring system having 5-30 aromatic ring atoms, which may be substituted by one or more non-aromatic radicals R³; two radicals Ar¹ here which are bonded to the same N atom or P atom may also be bridged to one another by a single bond or a bridge selected from N(R³), C(R³)₂, O or S;
R³ is selected on each occurrence, identically or differently, from the group consisting of H, D, F, Cl, Br, I, CN, NO₂, N(R⁴)₂, C(=O)R⁴, P(=O)(R⁴)₂, a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, which may in each case be substituted by one or more radicals R⁴, where one or more non-adjacent CH₂ groups may be replaced by R⁴C=CR⁴, C≡C, Si(R⁴)₂, C=O, C=S, C=NR⁴, P(=O)(R⁴), SO, SO₂, NR⁴, O, S or CONR⁴ and where one or more H atoms may be replaced by D, F, Cl, Br, I, CN or NO₂, an aromatic or heteroaromatic ring system having 5 to 60 aromatic ring atoms, which may in each case be substituted by one or more radicals R⁴, an aryloxy or heteroaryloxy group having 5 to 60 aromatic ring atoms, which may be substituted by one or more radicals R⁴, or a combination of these systems, where two or more adjacent substituents R³ may optionally form a monocyclic or polycyclic, aliphatic ring system, which may be substituted by one or more radicals R⁴;
R⁴ is selected on each occurrence, identically or differently, from the group consisting of H, D, F, CN, an aliphatic hydrocarbon radical having 1 to 20 C atoms, an aromatic or heteroaromatic ring system having 5 to 30 aromatic ring atoms, in which one or more H atoms may be replaced by D, F, Cl, Br, I or CN, where two or more adjacent substituents R⁴ may form a mono- or polycyclic, aliphatic ring system with one another;
with the proviso that at least one substituent R¹ which is selected from the group consisting of CN, N(Ar¹)₂, C(=O)Ar¹, P(=O)(Ar¹)₂, B(Ar¹)₂, Si(Ar¹)₃, Si(R³)₃ or an aromatic or heteroaromatic ring system having 5 to 60 aromatic ring atoms, which may in each case be substituted by one or more radicals R³, is present in the formulae (5a) to (5u).

2. Compound according to Claim 1, where the compound is selected from the formulae (5d) to (5u) in which one group R¹ is a hole-transporting unit and the other group R¹ is an electron-transporting unit.

3. Compound according to Claim 1 or 2, **characterised in that**, if R¹ stands for an aromatic or heteroaromatic ring system, this is selected, identically or differently on each occurrence, from benzene, ortho-, meta- or para-biphenyl, ortho-, meta-, para- or branched terphenyl, ortho-, meta- para- or branched quaterphenyl, 1- or 2-naphthyl, pyrrole, furan, thiophene, indole, benzofuran, benzothiophene, carbazole, dibenzofuran, dibenzothiophene, pyridine, pyrimidine, pyrazine, pyridazine, triazine, anthracene, phenanthrene, pyrene, benzanthracene or combinations of two or three of these groups, which may in each case be substituted by one or more radicals R³.

4. Compound according to one or more of Claims 1 to 3, **characterised in that** the compound, if it is used as electron-transport material or as matrix material for a phosphorescent emitter, is substituted by at least one group -C(=O)Ar¹ or -P(=O)(Ar¹)₂ or an electron-deficient heteroaromatic ring system, where preferably at least one substituent R¹ is selected from structures of the formulae (13) to (16), 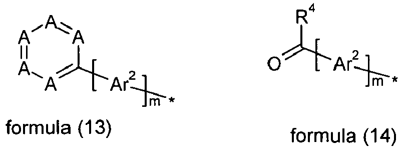 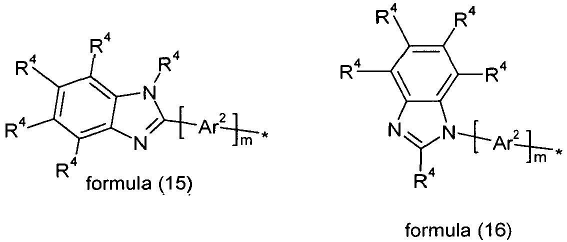 where R⁴ has the meaning given in Claim 1, * indicates the position of the bonding of the group of the formulae (13) to (16) and furthermore:
m is 0 or 1;
A is on each occurrence, identically or differently, CR⁴ or N, with the proviso that one, two or three groups A stand for N;
Ar² is, identically or differently on each occurrence, a divalent aromatic or heteroaromatic ring system having 5 to 16 C atoms, which may be substituted by one or more radicals R⁴.

5. Compound according to one or more of Claims 1 to 4, **characterised in that** the compound, if it is used as hole-transport material, as hole-injection material or as electron- or exciton-blocking material or as matrix material for a phosphorescent emitter, is substituted by at least one triarylamine derivative, carbazole derivative, indenocarbazole derivative, indolocarbazole derivative, azacarbazole derivative, indole derivative, furan derivative, benzofuran derivative, dibenzofuran derivative, thiophene derivative, benzothiophene derivative or dibenzothiophene derivative, which may in each case be substituted by one or more radicals R³, or at least one substituent R¹ stands for -N(Ar¹)₂, where these groups are preferably selected from the groups of the following formulae (35) to (49), 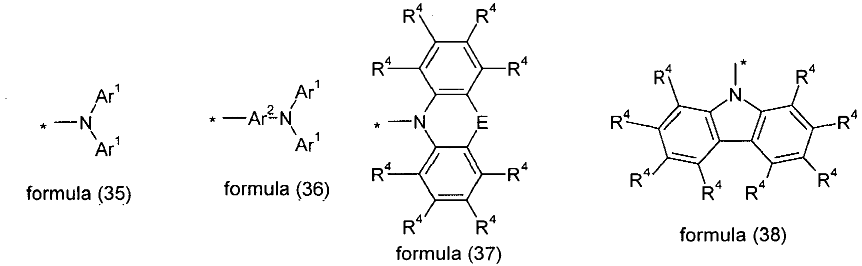 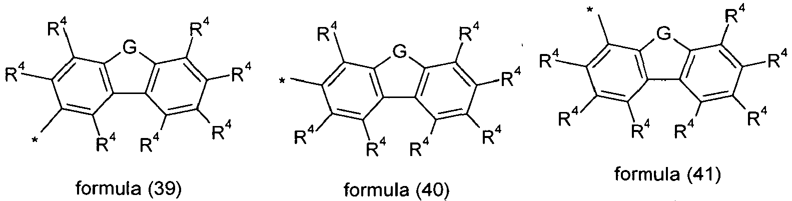 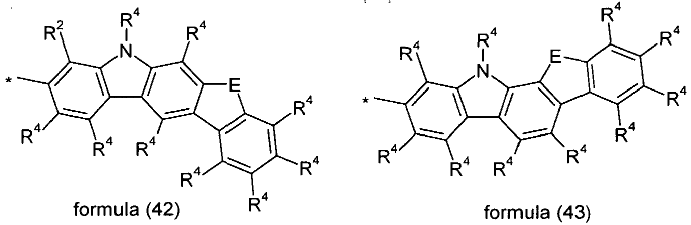 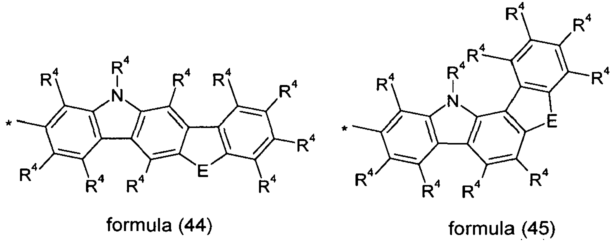 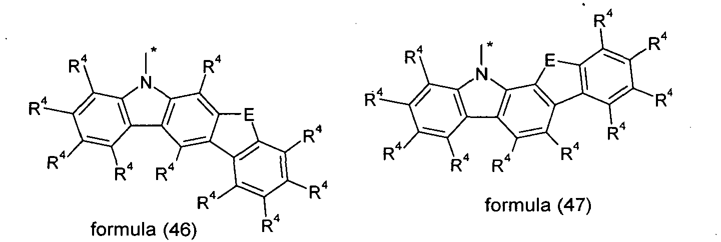 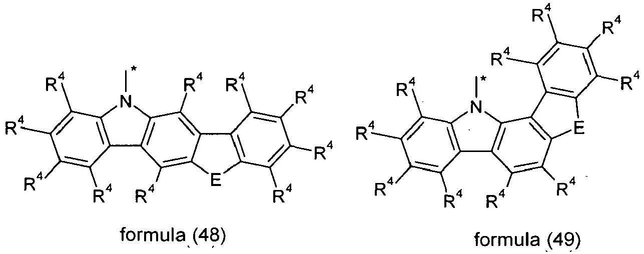 where the symbols used have the meanings given in Claims 1 and 4 and furthermore:
E is selected from the group consisting of C(R³)₂, NR³, O or S;
G is selected from the group consisting of NR³, O or S.

6. Process for the preparation of a compound according to one or more of Claims 1 to 5, comprising the reaction steps:
a) synthesis of a halogen-substituted spiro-9,9-bixanthene; and
b) reaction of the halogen-substituted spiro-9,9-bixanthene in a C-C coupling, C-N coupling, silylation, phosphanylation, boranylation or polycondensation.

7. Oligomer, polymer or dendrimer containing one or more compounds according to one or more of Claims 1 to 5, where one or more bonds from the compound to the polymer, oligomer or dendrimer are present at one or more positions instead of substituents.

8. Use of a compound according to one or more of Claims 1 to 5 or an oligomer, polymer or dendrimer according to Claim 7 in an electronic device, in particular in an organic electroluminescent device.

9. Electronic device containing at least one compound according to one or more of Claims 1 to 5 or an oligomer, polymer or dendrimer according to Claim 7, where the electronic device is selected, in particular, from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices.

10. Electronic device according to Claim 9, which is an organic electroluminescent device, **characterised in that** the compound according to one or more of Claims 1 to 5 is used as matrix material for fluorescent or phosphorescent emitters and/or as fluorescent emitter and/or in an electron-blocking or exciton-blocking layer and/or in a hole-transport layer and/or in a hole-injection layer and/or in a hole-blocking layer and/or in an electron-transport layer, depending on the precise substitution.

## Revendications

1. Composé selon l'une des formules (5a) à (5u) : 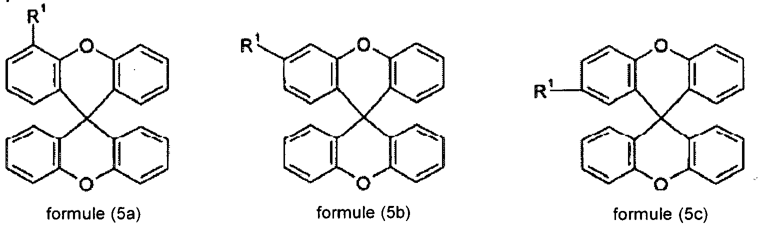 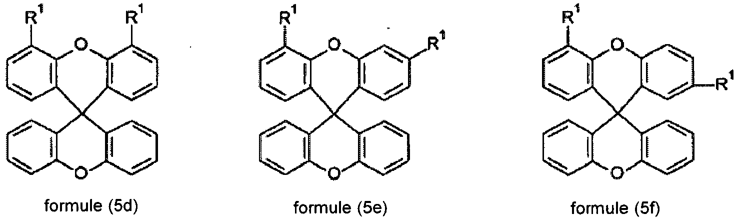 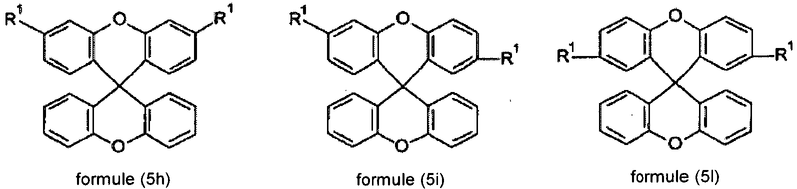 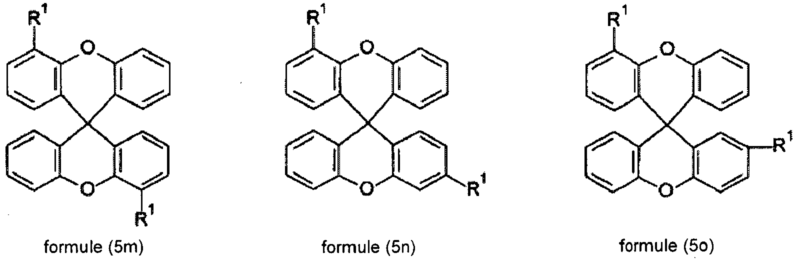 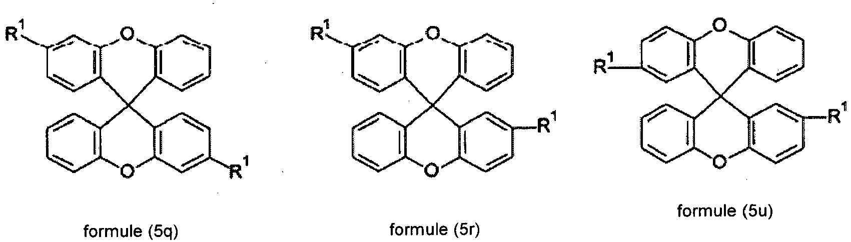 et dans lesquelles ce qui suit s'applique aux symboles et indices utilisés :
R¹ est sélectionné pour chaque occurrence, de manière identique ou différente, parmi le groupe qui est constitué par H, D, F, CI, Br, I, CN, NO₂, N(Ar¹)₂, N(R³)₂, C(=O)Ar¹, C(=O)R³, P(=O)(Ar¹)₂, B(Ar¹)₂, Si(Ar¹)₃, Si(R³)₃, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C ou un groupe alkényle ou alkynyle qui comporte 2 à 40 atomes de C, lequel peut, dans chaque cas, être substitué par un radical ou plusieurs radicaux R³, où un ou plusieurs groupe(s) CH₂ non adjacents peut/peuvent être remplacé(s) par R³C=CR³, C≡C, Si(R³)₂, C=O, C=S, C=NR³, P(=O)(R³), SO, SO₂, NR³, O, S ou CONR³ et où un ou plusieurs atome(s) de H peut/peuvent être remplacé(s) par D, F, CI, Br, I, CN ou NO₂, un système de cycle aromatique ou hétéroaromatique qui comporte 5 à 60 atomes de cycle aromatique, lequel peut, dans chaque cas, être substitué par un radical ou plusieurs radicaux R³, un groupe aryloxy ou hétéroaryloxy qui comporte 5 à 60 atomes de cycle aromatique, lequel peut être substitué par un radical ou plusieurs radicaux R³;
Ar¹ est pour chaque occurrence, de manière identique ou différente, un système de cycle aromatique ou hétéroaromatique qui comporte 5 à 30 atomes de cycle aromatique, lequel peut être substitué par un radical ou plusieurs radicaux non aromatique(s) R³ ; deux radicaux Ar¹ ici qui sont liés au même atome de N ou au même atome de P peuvent également être pontés l'un à l'autre au moyen d'une liaison simple ou d'un pont qui est sélectionné parmi N(R³), C(R³)₂, O ou S ;
R³ est sélectionné pour chaque occurrence, de manière identique ou différente, parmi le groupe qui est constitué par H, D, F, CI, Br, I, CN, NO₂, N(R⁴)₂, C(=O)R⁴, P(=O)(R⁴)₂, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C ou un groupe alkényle ou alkynyle qui comporte 2 à 40 atomes de C, lequel peut, dans chaque cas, être substitué par un radical ou plusieurs radicaux R⁴, où un ou plusieurs groupe(s) CH₂ non adjacents peut/peuvent être remplacé(s) par R⁴C=CR⁴, C≡C, Si(R⁴)₂, C=O, C=S, C=NR⁴, P(=O)(R⁴), SO, SO₂, NR⁴, O, S ou CONR⁴ et où un ou plusieurs atome(s) de H peut/peuvent être remplacé(s) par D, F, CI, Br, I, CN ou NO₂, un système de cycle aromatique ou hétéroaromatique qui comporte 5 à 60 atomes de cycle aromatique, lequel peut, dans chaque cas, être substitué par un radical ou plusieurs radicaux R⁴, un groupe aryloxy ou hétéroaryloxy qui comporte 5 à 60 atomes de cycle aromatique, lequel peut être substitué par un radical ou plusieurs radicaux R⁴, ou une combinaison de ces systèmes, où deux substituants R³ adjacents ou plus peuvent en option former un système de cycle aliphatique monocyclique ou polycyclique, lequel peut être substitué par un radical ou plusieurs radicaux R⁴ ;
R⁴ est sélectionné pour chaque occurrence, de manière identique ou différente, parmi le groupe qui est constitué par H, D, F, CN, un radical hydrocarbone aliphatique qui comporte 1 à 20 atome(s) de C, un système de cycle aromatique ou hétéroaromatique qui comporte 5 à 30 atomes de cycle aromatique, où un ou plusieurs atome(s) de H peut/peuvent être remplacé(s) par D, F, CI, Br, I ou CN, où deux substituants R⁴ adjacents ou plus peuvent former un système de cycle aliphatique monocyclique ou polycyclique l'un avec l'autre ou les uns avec les autres ;
étant entendu qu'au moins un substituant R¹ qui est sélectionné parmi le groupe qui est constitué par CN, N(Ar¹)₂, C(=O)Ar¹, P(=O)(Ar¹)₂, B(Ar¹)₂, Si(Ar¹)₃, Si(R³)₃ ou un système de cycle aromatique ou hétéroaromatique qui comporte 5 à 60 atomes de cycle aromatique, lequel peut, dans chaque cas, être substitué par un radical ou plusieurs radicaux R³, est présent dans les formules (5a) à (5u).

2. Composé selon la revendication 1, dans lequel le composé est sélectionné parmi les formules (5d) à (5u) dans lesquelles un groupe R¹ est une unité de transport de trous et l'autre groupe R¹ est une unité de transport d'électrons.

3. Composé selon la revendication 1 ou 2, **caractérisé en ce que**, si R¹ représente un système de cycle aromatique ou hétéroaromatique, celui-ci est sélectionné, de manière identique ou différente pour chaque occurrence, parmi benzène, ortho-biphényle, méta-biphényle ou para-biphényle, ortho-terphényle, méta-terphényle, para-terphényle ou terphényle ramifié, ortho-quaterphényle, méta-quaterphényle, para-quaterphényle ou quaterphényle ramifié, 1-naphtyle ou 2-naphtyle, pyrrole, furane, thiophène, indole, benzofurane, benzothiophène, carbazole, dibenzofurane, dibenzothiophène, pyridine, pyrimidine, pyrazine, pyridazine, triazine, anthracène, phénanthrène, pyrène, benzanthracène ou des combinaisons de deux ou trois de ces groupes, lesquels peuvent, dans chaque cas, être substitués par un radical ou plusieurs radicaux R³.

4. Composé selon une ou plusieurs des revendications 1 à 3, **caractérisé en ce que** le composé, s'il est utilisé en tant que matériau de transport d'électrons ou en tant que matériau de matrice pour un émetteur phosphorescent, est substitué par au moins un groupe -C(=O)Ar¹ ou -P(=O)(Ar¹)₂ ou un cycle hétéroaromatique déficient en électrons, où, de façon préférable, au moins un substituant R¹ est sélectionné parmi des structures des formules (13) à (16) : 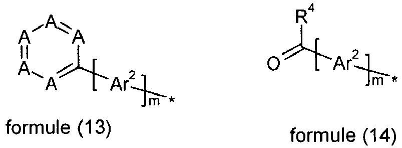 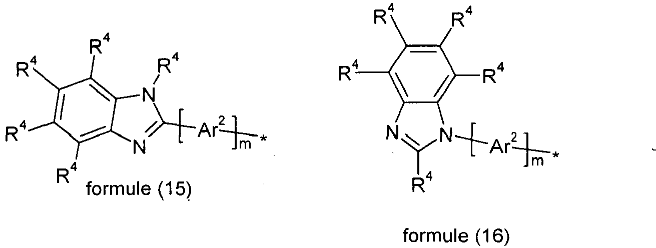 dans lesquelles R⁴ présente la signification qui est donnée selon la revendication 1, * indique la position de la liaison du groupe des formules (13) à (16) et en outre :
m est 0 ou 1 ;
A est, pour chaque occurrence, de manière identique ou différente, CR⁴ ou N, étant entendu qu'un, deux ou trois groupe(s) A représente(nt) N ;
Ar² est, de manière identique ou différente pour chaque occurrence, un système de cycle divalent aromatique ou hétéroaromatique qui comporte 5 à 16 atomes de C, lequel peut être substitué par un radical ou plusieurs radicaux R⁴.

5. Composé selon une ou plusieurs des revendications 1 à 4, **caractérisé en ce que** le composé, s'il est utilisé en tant que matériau de transport de trous, en tant que matériau d'injection de trous ou en tant que matériau de blocage d'électrons ou d'excitons ou en tant que matériau de matrice pour un émetteur phosphorescent, est substitué par au moins un dérivé de triarylamine, un dérivé de carbazole, un dérivé d'indénocarbazole, un dérivé d'indolocarbazole, un dérivé d'azacarbazole, un dérivé d'indole, un dérivé de furane, un dérivé de benzofurane, un dérivé de dibenzofurane, un dérivé de thiophène, un dérivé de benzothiophène ou un dérivé de dibenzothiophène, lequel peut, dans chaque cas, être substitué par un radical ou plusieurs radicaux R³, ou au moins un substituant R¹ représente -N(Ar¹)₂, où ces groupes sont de façon préférable sélectionnés parmi les groupes des formules (35) à (49) qui suivent : 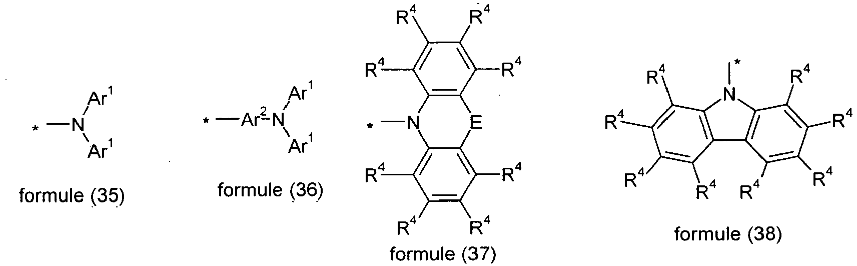 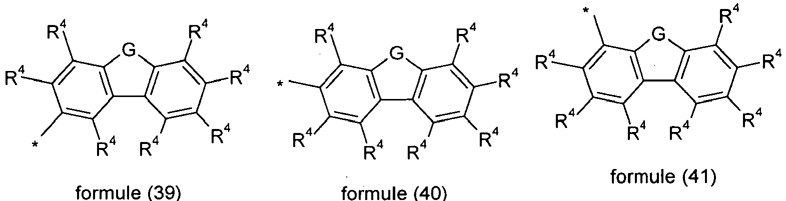 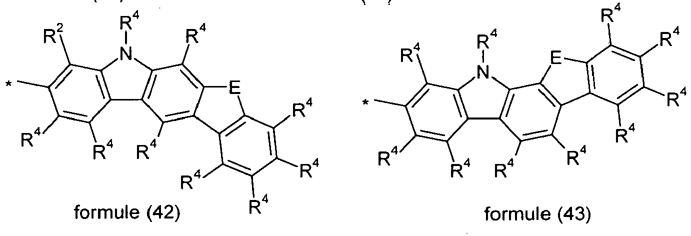 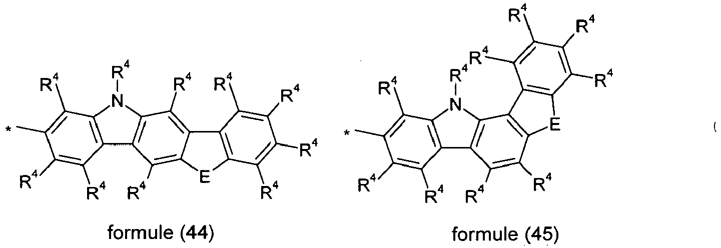 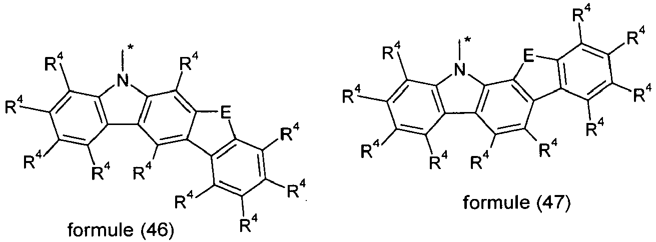 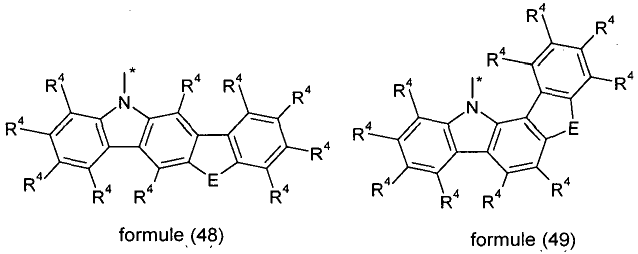 dans lesquelles les symboles utilisés présentent les significations qui sont données selon les revendications 1 et 4 et en outre :
E est sélectionné parmi le groupe qui est constitué par C(R³)₂, NR³, O ou S ;
G est sélectionné parmi le groupe qui est constitué par NR³, O ou S.

6. Procédé pour la préparation d'un composé selon une ou plusieurs des revendications 1 à 5, comprenant les étapes de réaction qui suivent :
a) synthèse d'un spiro-9,9-bixanthène substitué par halogène ; et
b) réaction du spiro-9,9-bixanthène substitué par halogène selon un couplage C-C, un couplage C-N, une silylation, une phosphanylation, une boranylation ou une polycondensation.

7. Oligomère, polymère ou dendrimère contenant un ou plusieurs composé(s) selon une ou plusieurs de revendications 1 à 5, où une ou plusieurs liaison(s) depuis le composé sur le polymère, l'oligomère ou le dendrimère est/sont présente(s) au niveau d'une ou de plusieurs position(s) en lieu et place de substituants.

8. Utilisation d'un composé selon une ou plusieurs de revendications 1 à 5 ou d'un oligomère, d'un polymère ou d'un dendrimère selon la revendication 7 dans un dispositif électronique, en particulier dans un dispositif électroluminescent organique.

9. Dispositif électronique contenant au moins un composé selon une ou plusieurs des revendications 1 à 5 ou un oligomère, un polymère ou un dendrimère selon la revendication 7, où le dispositif électronique est sélectionné, en particulier, parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires organiques sensibilisées par colorant, les détecteurs optiques organiques, les photorécepteurs organiques, les dispositifs à extinction de champ organiques, les cellules électrochimiques émettrices de lumière, les diodes laser organiques et les dispositifs à émission de plasmons organiques.

10. Dispositif électronique selon la revendication 9, lequel est un dispositif électroluminescent organique, **caractérisé en ce que** le composé selon une ou plusieurs de revendications 1 à 5 est utilisé en tant que matériau de matrice pour des émetteurs fluorescents ou phosphorescents et/ou en tant qu'émetteur fluorescent et/ou dans une couche de blocage d'électrons ou de blocage d'excitons et/ou dans une couche de transport de trous et/ou dans une couche d'injection de trous et/ou dans une couche de blocage de trous et/ou dans une couche de transport d'électrons, en fonction de la substitution précise.
